# EUROPEAN PATENT SPECIFICATION

(11) **EP 2 204 447 B1**
(45) Date of publication and mention of the grant of the patent: **01.06.2016**
(21) Application number: 09180683.6
(22) Date of filing: 08.10.1999
(51) Int. Cl.: C12N 15/10, C12N 5/10, C07K 16/00, C12Q 1/68, A01K 67/027

(54) **Method for generating diversity**
Methode zur Diversitätsbildung
Méthode de génération de diversité

(30) Priority: 09.10.1998 GB 9822104; 19.01.1999 GB 9901141; 09.06.1999 GB 9913435
(43) Date of publication of application: 07.07.2010
(62) Divisional of application: 99947775.5
(73) Proprietor: Medical Research Council, Swindon Wiltshire SN2 1FL (GB)
(72) Inventor: Sale, Julian Edward, Cambridge CB2 2QH (GB); Neuberger, Michael,S., Cambridge CB2 2QH (GB); Cumbers, Sarah Jane, Coulsdon CR5 2NR (GB)
(74) Representative: D Young & Co LLP

(56) References cited:
- WO-A-93/12228
- WO-A-95/12689
- US-A- 5 750 335
- J.E. SALE AND M.S. NEUBERGER: "TdT-Accessible breaks are scattered over the immunoglobulin V domain in a constitutively hypermutating B cell line", IMMUNITY, vol. 9, no. 6, December 1998 (1998-12), pages 859-869, XP000876807, CELL PRESS, CAMBRIDGE, MA, US
- C.J. JOLLY ET AL.: "Rapid methods for the analysis of immunglobulin gene hypermutation: application to transgenic and gene targeted mice", NUCLEIC ACIDS RESEARCH, vol. 25, no. 10, 1997, pages 1913-1919, XP002130744, IRL PRESS LIMITED,OXFORD,ENGLAND
- M. MU-QUAN LIN ET AL.: "Sequence dependent hypermutation of the immunoglobulin heavy chain in cultured B cells", PROC. NATL. ACAD. SCI., vol. 94, May 1997 (1997-05), pages 5284-5289, XP002130747, NATL. ACAD. SCI.,WASHINGTON,DC,US;
- S. DENÉPOUX ET AL.: "Induction of somatic mutation in a human B cell line in vitro", IMMUNITY, vol. 6, January 1997 (1997-01), pages 35-46, XP000876832, CELL PRESS, CAMBRIDGE, MA, US
- GREEN N S ET AL: "Ig V region hypermutation in B cell hybrids mimics in vivo mutation and allows for isolation of clonal variants", MOLECULAR IMMUNOLOGY, PERGAMON, GB, vol. 34, no. 15, 1 October 1997 (1997-10-01), pages 1095-1103, XP002962726, ISSN: 0161-5890, DOI: DOI:10.1016/S0161-5890(97)00131-4
- GREEN N S ET AL: "Immunoglobulin hypermutation in cultured cells", IMMUNOLOGICAL REVIEWS 199804 DK, vol. 162, April 1998 (1998-04), pages 77-87, XP002631042, ISSN: 0105-2896
- YELAMOS J ET AL: "Targeting of non-lg sequences in place of the V segment by somatic hypermutation", NATURE, NATURE PUBLISHING GROUP, LONDON, GB, vol. 376, no. 6537, 1 January 1995 (1995-01-01), pages 225-229, XP002211125, ISSN: 0028-0836, DOI: DOI:10.1038/376225A0
- E. KÄLLBERG ET AL.: "Somatic mutation of immunglobulin V genes in vitro", SCIENCE, vol. 271, 1 March 1996 (1996-03-01), pages 1285-1289, XP002130746, AAAS,WASHINGTON,DC,US
- N. KLIX ET AL.: "Mulitiple sequences from downstream of the J-kappa cluster can combine to recruit somatic hypermutation to a heterologous, upstream mutation domain", EUROPEAN J. IMMUNOLOGY, vol. 28, January 1998 (1998-01), pages 317-326, XP000876803, VCH VERLAGSGESELLSCHAFT MBH, WEINHEIM, BRD
- M.J. SHARPE ET AL.: "Somatic hypermutation of immunglobulin kappa may depend on sequences 3' of c-kappa and occurs on passenger transgenes", EMBO J., vol. 10, no. 8, August 1991 (1991-08), pages 2139-2145, XP002130745, OXFORD UNIVERSITY PRESS,GB;
- GREEN N S ET AL: 'Immunoglobulin variable region hypermutation in hybrids derived from a pre-B- and a myeloma cell line.' PROCEEDINGS OF THE NATIONAL ACADEMY OF SCIENCES OF THE UNITED STATES OF AMERICA vol. 92, no. 14, 03 July 1995, pages 6304 - 6308 ISSN: 0027-8424

## Description

The present invention relates to a method for generating diversity in a gene or gene product by exploiting the natural somatic hypermutation capability of antibody-producing cells, as well as to cell lines capable of generating diversity in defined gene products.

Many *in vitro* approaches to the generation of diversity in gene products rely on the generation of a very large number of mutants which are then selected using powerful selection technologies. For example, phage display technology has been highly successful as providing a vehicle that allows for the selection of a displayed protein (Smith, 1985; Bass *et al.,* 1990; McCafferty *et al.,* 1990; for review see Clackson and Wells, 1994) Similarly, specific peptide ligands have been selected for binding to receptors by affinity selection using large libraries of peptides linked to the C terminus of the lac repressor Lacl (Cull *et al.,* 1992). When expressed in *E. coli* the repressor protein physically links the ligand to the encoding plasmid by binding to a lac operator sequence on the plasmid. Moreover, an entirely *in vitro* polysome display system has also been reported (Mattheakis *et al.,* 1994) in which nascent peptides are physically attached via the ribosome to the RNA which encodes them.

*In vivo* the primary repertoire of antibody specificities is created by a process of DNA rearrangement involving the joining of immunoglobulin V, D and J gene segments. Following antigen encounter in mouse and man, the rearranged V genes in those B cells that have been triggered by the antigen are subjected to a second wave of diversification, this time by somatic hypermutation. This hypermutation generates the secondary repertoire from which good binding specificities can be selected thereby allowing affinity maturation of the humoral immune response.

Artificial selection systems to date rely heavily on initial mutation and selection, similar in concept to the initial phase of V-D-J rearrangement which occurs in natural antibody production, in that it results in the generation of a "fixed" repertoire of gene product mutants from which gene products having the desired activity may be selected

*In vitro* RNA selection and evolution (Ellington and Szostak, 1990), sometimes referred to as SELEX (systematic evolution of ligands by exponential enrichment) (Tuerk and Gold, 1990) allows for selection for both binding and chemical activity, but only for nucleic acids. When selection is for binding, a pool of nucleic acids is incubated with immobilised substrate. Non-binders are washed away, then the binders are released, amplified and the whole process is repeated in iterative steps to enrich for better binding sequences. This method can also be adapted to allow isolation of catalytic RNA and DNA (Green and Szostak, 1992; for reviews see Chapman and Szostak, 1994; Joyce, 1994; Gold *et* al., 1995; Moore, 1995). SELEX, thus, permits cyclical steps of improvement of the desired activity, but is limited in its scope to the preparation of nucleic acids.

Unlike in the natural immune system, however, artificial selection systems are poorly suited to any facile form of "affinity maturation", or cyclical steps of repertoire generation and development. One of the reasons for this is that it is difficult to target mutations to regions of the molecule where they are required, so subsequent cycles of mutation and selection do not lead to the isolation of molecules with improved activity with sufficient efficiency.

Much of what is known about the somatic hypermutation process which occurs during affinity maturation in natural antibody production has been derived from an analysis of the mutations that have occurred during hypermutation *in vivo* (for reviews see Neuberger and Milstein, 1995; Weill and Raynaud, 1996; Parham, 1998). Most of these mutations are single nucleotide substitutions which are introduced in a stepwise manner. They are scattered over the rearranged V domain, though with characteristic hotspots, and the substitutions exhibit a bias for base transitions. The mutations largely accumulate during B cell expansion in germinal centres (rather than during other stages of B cell differentiation and proliferation) with the rate of incorporation of nucleotide substitutions into the V gene during the hypermutation phase estimated at between 10⁻⁴ and 10⁻³ bp⁻¹ generation⁻¹ (McKean et al., 1984; Berek & Milstein, 1988)

The possibility that lymphoid cell lines could provide a tractable system for investigating hypermutation was considered many years ago (Coffino and Scharff, 1971; Adetugbo et al., 1977; Brüggemann et al., 1982). Clearly, it is important that the rate of V gene mutation in the cell-line under study is sufficiently high not only to provide a workable assay but also to be confident that mutations are truly generated by the localised antibody hypermutation mechanism rather than reflecting a generally increased mutation rate as is characteristically associated with many tumours. Extensive studies on mutation have been performed monitoring the reversion of stop codons in V_{H} in mouse pre-B and plasmacytoma cell lines (Wabl et al., 1985; Chui et al., 1995; Zhu et al., 1995; reviewed by Green et al., 1998). The alternative strategy of direct sequencing of the expressed V gene has indicated that V_{H} gene diversification in several follicular, Burkitt and Hodgkin lymphomas can continue following the initial transformation event (Bahler and Levy, 1992; Jain et al., 1994; Chapman et al., 1995 and 1996; Braeuninger et al., 1997). Direct sequencing has also revealed a low prevalence of mutations in a cloned follicular lymphoma line arguing that V_{H} diversification can continue in vitro (Wu et al., 1995). None of the reports of constitutive mutation in cell lines cited above provides evidence that the mutations seen are the result of directed hypermutation, as observed in natural antibody diversification, which is concentrated in the V genes, as opposed to a general susceptibility to mutation as described in many tumour cell lines from different lineages.

Recently, hypermutation has been induced in a cell line by Denepoux *et al.* (1997), by culturing cells in the presence of anti-immunoglobulin antibody and activated T-cells. However, the hypermutation observed was stated to be induced, not constitutive,

Sale JE and Neuberger MS (Immunity 1998 Dec;9(6):859-69) describe an IgM-expressing Burkitt lymphoma line that constitutively diversifies its immunoglobulin V domain at high rate during culture.

Jolly CJ et al. (Nucleic Acids Res. 1997 May 15;25(10):1913-9) describe methods for the analysis of immunoglobulin gene hypermutation and their application to transgenic and gene targeted mice.

Lin M et al. (Proc Natl Acad Sci USA. 1997 May 13;94(10):5284-9) describe sequence dependent hypermutation of the immunoglobulin heavy chain in cultured B cells.

Denépoux S et al. (Immunity. 1997 Jan;6(1):35-46) describe the induction of somatic mutation in a human B cell line in vitro.

Green NS et al. (Mol Immunol. 1997 Oct;34(15):1095-103) describe a cell culture system in which reversion of a V region stop codon in a stably transfected Ig gene permits the quantitation of mutation rates by fluctuation analysis.

Green NS et al. (Immunological Reviews 1998 Apr;162(1):77-87) describe immunoglobulin hypermutation in cultured cells.

Yélamos J et al. (Nature. 1995 Jul 20;376(6537):225-9) describe the targeting of non-Ig sequences in place of the V segment by somatic hypermutation.

Källberg E et al. (Science. 1996 Mar 1;271(5253):1285-9) describe the somatic mutation of immunoglobulin V genes in vitro.

### Summary of the Invention

The present invention provides the use of an immunoglobulin-expressing eukaryotic cell in an *in vitro* method of directed constitutive hypermutation of one or more nucleic acid sequences in the preparation and isolation of a gene product having a desired activity, wherein the one or more nucleic acid sequences encoding the gene product are operatively linked to control sequences which direct somatic hypermutation of the one or more nucleic acids encoding the gene product without the requirement for external stimulation to produce one or more mutated nucleic acid sequences, wherein the one or more mutated nucleic acid sequences encode a gene product having a desired activity, as specified in the claims.

In a first aspect of the present disclosure there is described a method for preparing a lymphoid cell line capable of directed constitutive hypermutation of a target nucleic acid region, comprising screening a cell population for ongoing target sequence diversification, and selecting a cell in which the rate of target nucleic acid mutation exceeds that of other nucleic acid mutation by a factor of 100 or more.

As used herein, "directed constitutive hypermutation" refers to the ability, observed for the first time in experiments reported herein, of certain cell lines to cause alteration of the nucleic acid sequence of one or more specific sections of endogenous or transgene DNA in a constitutive manner, that is without the requirement for external stimulation. In cells capable of directed constitutive hypermutation, sequences outside of the specific sections of endogenous or transgene DNA are not subjected to mutation rates above background mutation rates.

A "target nucleic acid region" is a nucleic acid sequence or region in the cell according to the invention which is subjected to directed constitutive hypermutation. The target nucleic acid may comprise one or more transcription units encoding gene products, which may be homologous or heterologous to the cell. Exemplary target nucleic acid regions are immunoglobulin V genes as found in immunoglobulin-producing cells These genes are under the influence of hypermutation-recruiting elements, as described further below, which direct the hypermutation to the locus in question. Other target nucleic acid sequences may be constructed, for example by replacing V gene transcription units in loci which contain hypermutation-recruiting elements with another desired transcription unit, or by constructing artificial genes comprising hypermutation-recruiting elements.

"Hypermutation" refers to the mutation of a nucleic acid in a cell at a rate above background. Preferably, hypermutation refers to a rate of mutation of between 10⁻⁵ and 10⁻³ bp⁻¹ generation⁻¹. This is greatly in excess of background mutation rates, which are of the order of 10⁻⁹ to 10⁻¹⁰ mutations bp⁻¹ generation⁻¹ (Drake *et al.,* 1988) and of spontaneous mutations observed in PCR. 30 cycles of amplification with Pfu polymerase would produce <0.05x10⁻³ mutations bp⁻¹ in the product, which in the present case would account for less than 1 in 100 of the observed mutations (Lundberg *et al.,* 1991)

Hypermutation is a part of the natural generation of immunoglobulin variable chain (V) genes. According to the present invention therefore, the cell line is preferably an immunoglobulin-producing cell line which is capable of producing at least one immunoglobulin V gene. A V gene may be a variable light chain (V₁) or variable heavy chain (V_{H}) gene, and may be produced as part of an entire immunoglobulin molecule; it may be a V gene from an antibody, a T-cell receptor or another member of the immunoglobulin superfamily. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). Thus, preferred cell lines according to the invention are derived from B-cells. According to the present invention, it has been determined that cell lines derived from antibody-producing B cells may be isolated which retain the ability to hypermutate V region genes, yet do not hypermutate other genes.

In a preferred embodiment, the cells according to the invention are derived from or related to cells which hypermutate *in vivo.* Cells which hypermutate *in vivo* are, for example, immunoglobulin-expressing cells, such as B-cells. Lymphoma cells, which are Ig-expressing cell tumours, are particularly good candidates for the isolation of constitutively hypermutating cell lines according to the present invention.

As used herein, "screening for ongoing target sequence diversification" refers to the determination of the presence of hypermutation in the target nucleic acid region of the cell lines being tested. This can be performed in a variety of ways, including direct sequencing or indirect methods such as the MutS assay (Jolly *et al.,* 1997) or monitoring the generation of immunoglobulin loss variants. Cells selected according to this procedure are cells which display target sequence diversification.

The cell population which is subjected to selection by the method of the disclosure may be a polyclonal population, comprising a variety of cell types and/or a variety of target sequences, or a (mono-)clonal population of cells.

A clonal cell population is a population of cells derived from a single clone, such that the cells would be identical save for mutations occurring therein. Use of a clonal cell population preferably excludes co-culturing with other cell types, such as activated T-cells, with the aim of inducing V gene hypermutation.

Cells according to the invention do not rely on the use of induction steps in order to produce hypermutation.

Preferably, the clonal cell population screened in the present disclosure is derived from a B cell. Advantageously it is a lymphoma cell line, such as a Burkitt lymphoma cell line, a follicular lymphoma cell line or a diffuse large cell lymphoma cell line.

Preferably, the method according to the disclosure further comprises the steps of isolating one or more cells which display target sequence diversification, and comparing the rate of accumulation of mutations in the target sequences with that in non-target sequences in the isolated cells.

A feature of the present disclosure is that the hypermutation is directed only to specific (target) nucleic acid regions, and is not observed outside of these regions in a general manner. Specificity is thus assayed as part of the method of the disclosure by assaying the rate of mutation of sequences other than target sequences. C region genes, which are not naturally exposed to hypermutation, may advantageously be employed in such a technique, although any other nucleic acid region not subject to specific hypermutation may also be used. Since hypermutation is not sequence dependent, the actual sequence of the nucleic acid region selected for comparison purposes is not important. However, it must not be subject to control sequences which direct hypermutation, as described below. Conveniently, background mutation may be assessed by fluctuation analysis, for example at the HPRT locus [see Luria and Delbreck., (1943); Capizzi and Jameson, (1973)].

Cells in which target region mutation exceeds non-target region mutation are cells capable of directed constitutive hypermutation of a specific nucleic acid region in accordance with the present invention. The factor by which V region gene mutation exceeds other gene mutation is variable, but is in general of the order of at least 10²' advantageously 10³, and preferably 10⁴ or more.

Overall mutation rates and diversity may be increased, for example by the administration of mutagens or expression of sequence codifying genes, such as terminal deoxynucleotidyl transferase (TdT). However, the difference between hypermutation and background is not expected to be increased in such a manner.

In a second aspect of the present disclosure, there is described a method for preparing a gene product having a desired activity, comprising the steps of:
a) expressing a nucleic acid encoding the gene product in a population of cells according to the first aspect of the present invention, operably linked to a nucleic acid which directs hypermutation;
b) identifying a cell or cells within the population of cells which expresses a mutant gene product having the desired activity; and
c) establishing one or more clonal populations of cells from the cell or cells identified in step (b), and selecting from said clonal populations a cell or cells which expresses a gene product having an improved desired activity.

The population of cells according to part a) above is derived from a clonal or polyclonal population of cells which comprises cells identified by a method according to the first aspect of the disclosure as being capable of constitutive hypermutation of V region genes. The gene product may thus be the endogenous immunoglobulin polypeptide, a gene product expressed by a manipulated endogenous gene or a gene product expressed by a heterologous transcription unit operatively linked to control sequences which direct somatic hypermutation, as described further below.

The nucleic acid which is expressed in the cells of the disclosure and subjected to hypermutation may be an endogenous region, such as the endogenous V region, or a heterologous region inserted into the cell line of the invention. This may take form, for example, of a replacement of the endogenous V region with heterologous transcription unit(s), such as a heterologous V region, retraining the endogenous control sequences which direct hypermutation; or of the insertion into the cell of a heterologous transcription unit under the control of its own control sequences to direct hypermutation, wherein the transcription unit may encode V region genes or any other desired gene product. The nucleic acid according to the disclosure is described in more detail below.

In step b) above, the cells are screened for the desired gene product activity. This may be, for example in the case of immunoglobulins, a binding activity. Other activities may also be assessed, such as enzymatic activities or the like, using appropriate assay procedures. Where the gene product is displayed on the surface of the cell, cells which produce the desired activity may be isolated by detection of the activity on the cell surface, for example by fluorescence, or by immobilising the cell to a substrate via the surface gene product. Where the activity is secreted into the growth medium, or otherwise assessable only for the entire cell culture as opposed to in each individual cell, it is advantageous to establish a plurality of clonal populations from step a) in order to increase the probability of identifying a cell which secretes a gene product having the desired activity. Advantageously, the selection system employed does not affect the cell's ability to proliferate and mutate.

Preferably, at this stage (and in step c) cells which express gene products having a better, improved or more desirable activity are selected. Such an activity is, for example, a higher affinity binding for a given ligand, or a more effective enzymatic activity. Thus, the method allows for selection of cells on the basis of a qualitative and/or quantitative assessment of the desired activity.

In a third aspect of the present disclosure, there is provided the use of a cell capable of directed constitutive hypermutation of a specific nucleic acid region in the preparation of a gene product having a desired activity.

In the use according to the disclosure, a nucleic acid encoding the gene product having the desired activity is operatively linked to control sequences which direct hypermutation within the cell. Successive generations of the cell thus produce mutants of the nucleic acid sequence, which are screened by the method of the disclosure to isolate mutants with advantageous properties.

### Brief Description of the Figures

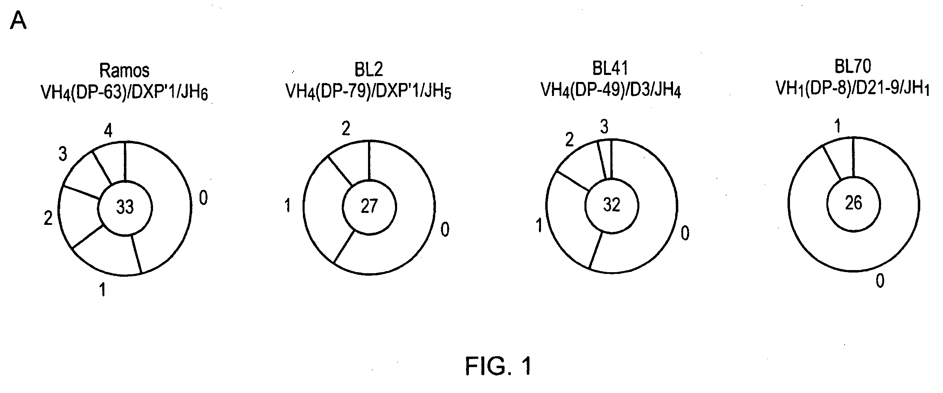

Figure 1 V_{H} diversity in Burkitt lines.
   (A) Sequence diversity in the rearranged V_{H} genes of four sporadic Burkitt lymphoma lines, shown as pie charts. The number of M13 clones sequenced for each cell line is denoted in the centre of the pie; the sizes of the various segments depict the proportion of sequences that are distinguished by 0, 1, 2 etc. mutations (as indicated) from the consensus.
   (B) Presumed dynastic relationship of V_{H} mutations identified in the initial Ramos culture. Each circle (with shading proportional to extent of mutation) represents a distinct sequence with the number of mutations accumulated indicated within the circle.
   (C) Mutation prevalence in the rearranged V_{λ} genes. Two V_{λ} rearrangements are identified in Ramos. Diversity and assignment of germline origin is presented as in
Figure 1A.
   (D) Comparison of mutation prevalence in the V_{H} and Cµ regions of the initial Ramos culture. Pie charts are presented as in Figure 1A.
Figure 2 Constitutive V_{H} diversification in Ramos.
   (A) Diversification assessed by a MutS assay. The mutation prevalence in each population as deduced by direct cloning and sequencing is indicated.
   (B) Dynastic relationships deduced from the progeny of three independent Ramos clones.
Figure 3 Distribution of unselected nucleotide substitutions along the Ramos V_{H}.
Figure 4. Hypermutation in Ramos generates diverse revertible IgM-loss variants.
   (A) Scheme showing the isolation of IgM-loss variants.
   (B) Table showing that multiple nonsense mutations can contribute to V_{H} inactivation. Each V_{H} codon position at which stops are observed in these two populations is listed.
   (C) Table of reversion rates of IgM-loss variants.
   (D) Sequence surrounding the stop codons in the IgM-loss derivatives.

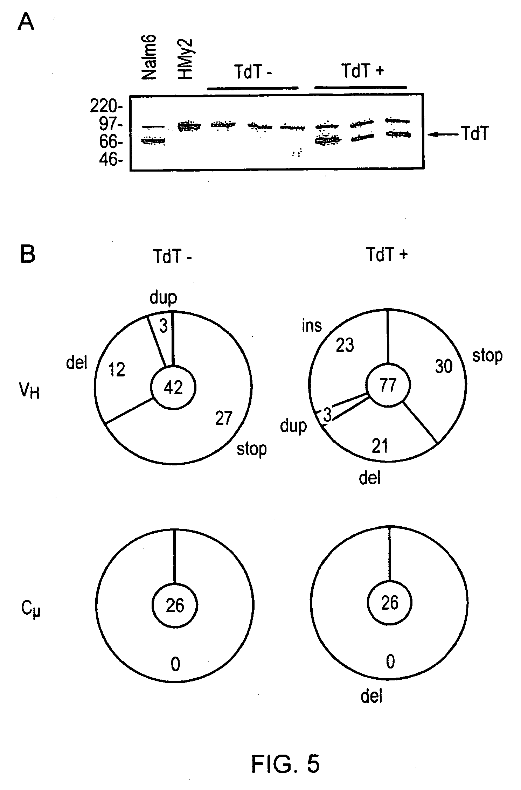

Figure 5. IgM-loss variants in Ramos transfectants expressing TdT.
   (A) Western blot analysis of expression of TdT in three pSV-pβG/TdT and three control transfectants of Ramos.
   (B) Pie charts depicting independent mutational events giving rise to IgM-loss variants.

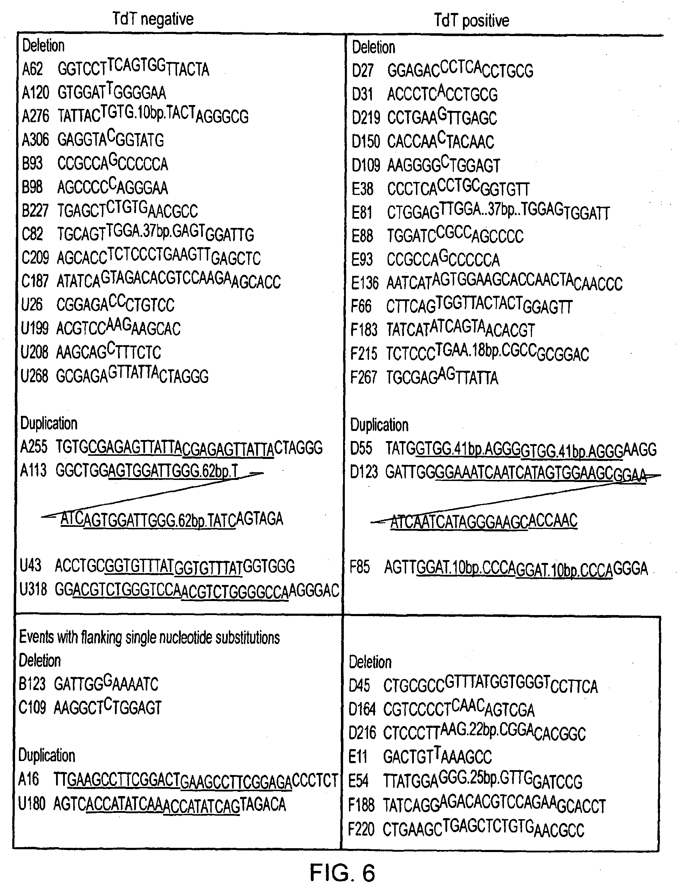

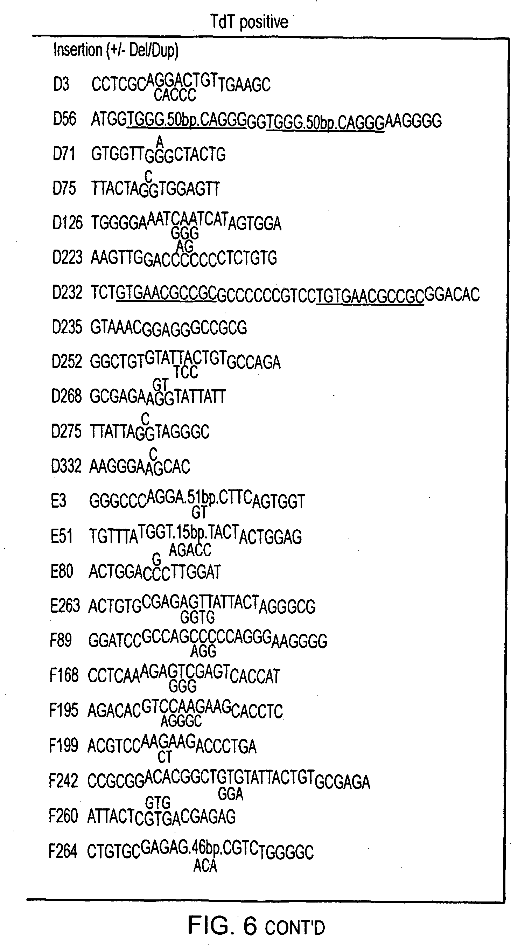

Figure 6. Sequence table summarising mutations in V_{H} other than single nucleotide substitutions.

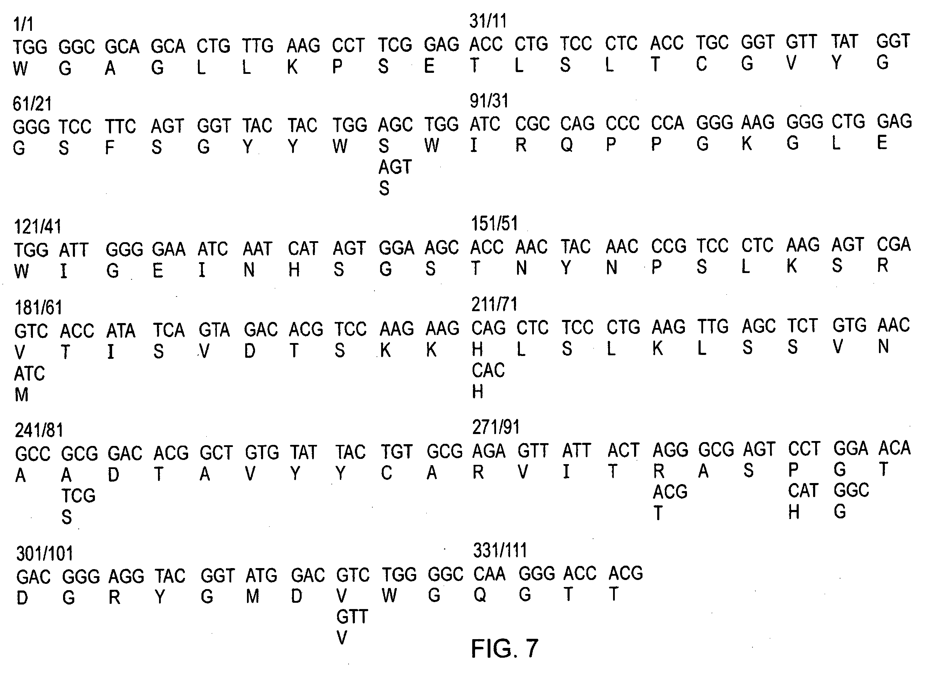

Figure 7. Comparison of sequences isolated from V_{H} genes of Ramos cells which have lost anti-idiotype (anti-Id1) binding specificity. Nucleotide substitutions which differ from the starting population consensus are shown in bold. Predicted amino acid changes are indicated, also in bold type.

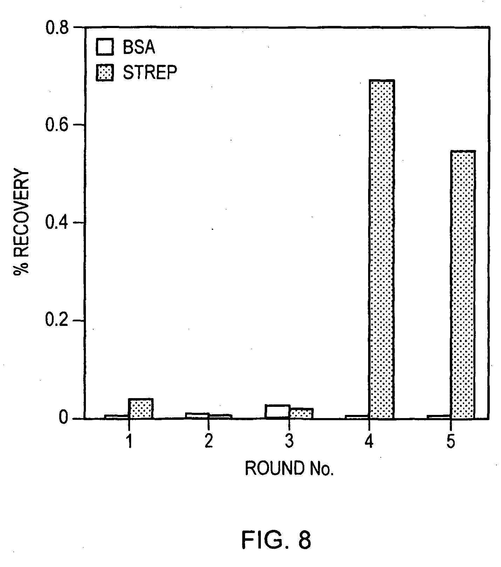

Figure 8. Bar graph showing enrichment of Ramos cells for production of an immunoglobulin with a novel binding specificity, by iterative selection over five rounds.

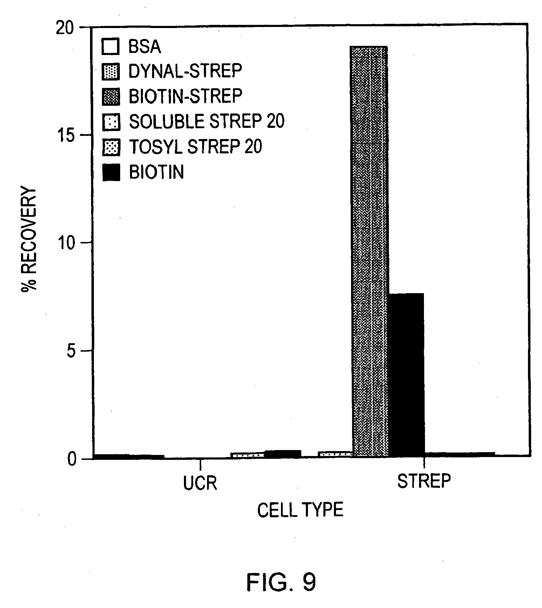

Figure 9. Bar graph showing improved recovery of Ramos cells binding a novel specificity (streptavidin) by increasing the bead:cell ratio.

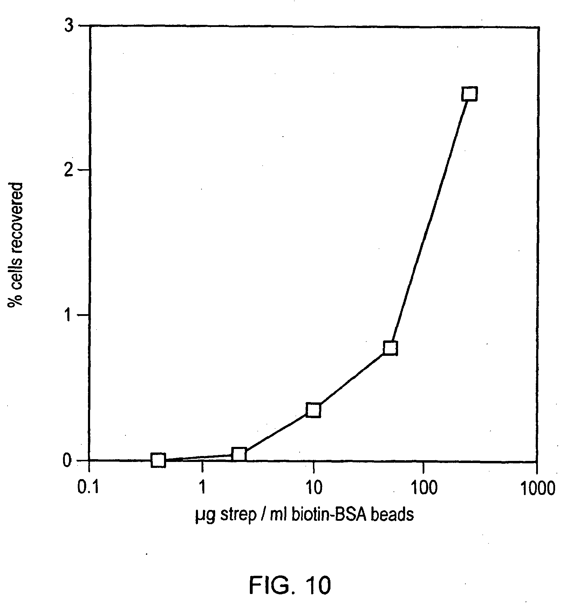

Figure 10. Chart showing increase in recovery of novel binding specificity Ramos cells according to increasing target antigen concentration.

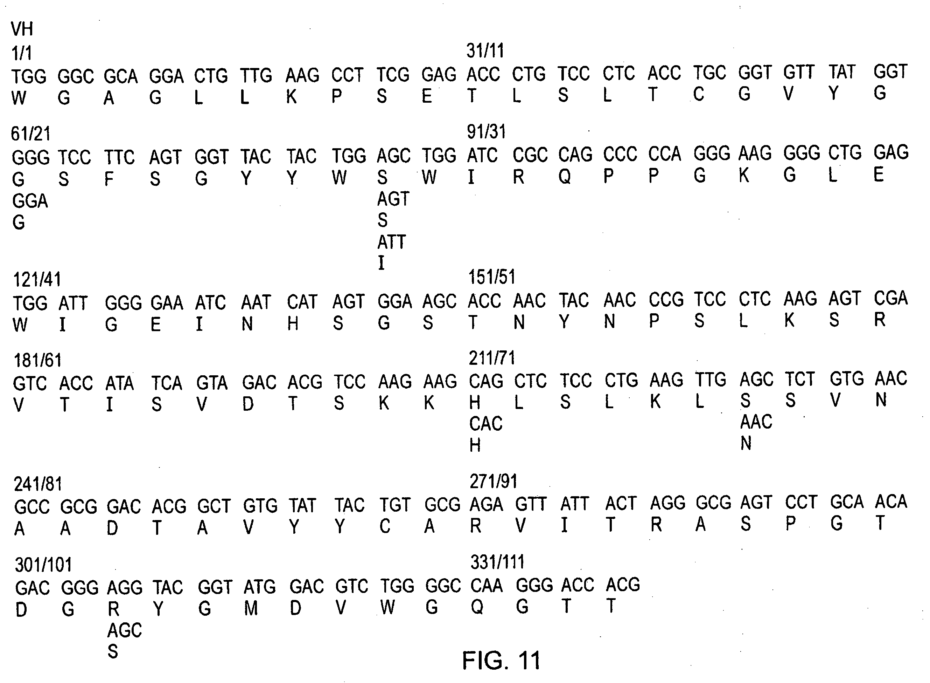

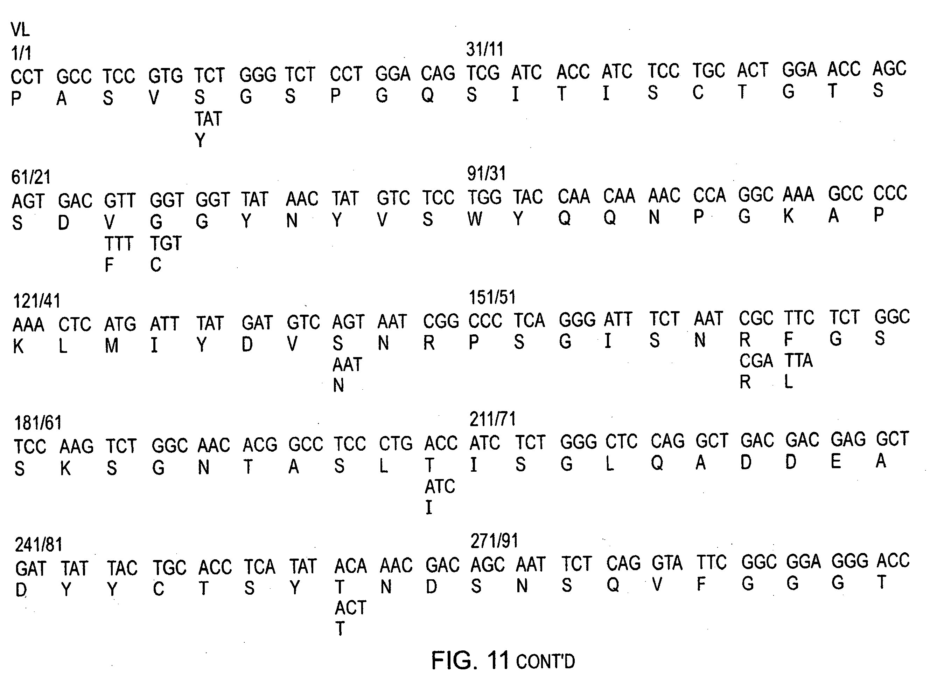

Figure 11. V_{H} sequence derived from streptavidin-binding Ramos cells. Nucleotide changes observed in comparison with the V_{H} sequence of the starting population, and predicted amino acid changes, are shown in bold.

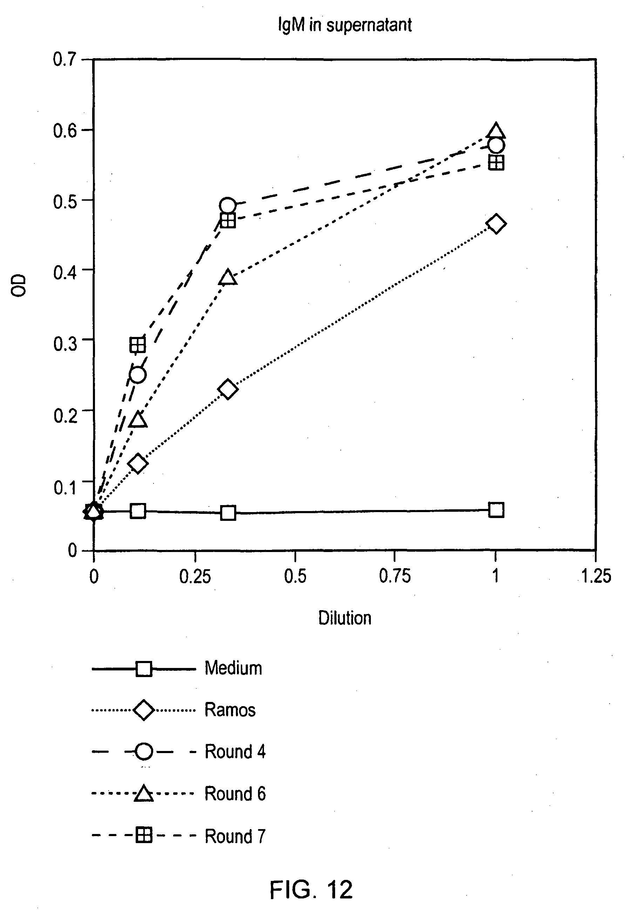

Figure 12. Amount of IgM in supernatants of cells selected in rounds 4, 6 and 7 of a selection process for streptavidin binding, against control medium and unselected Ramos cell supernatant.

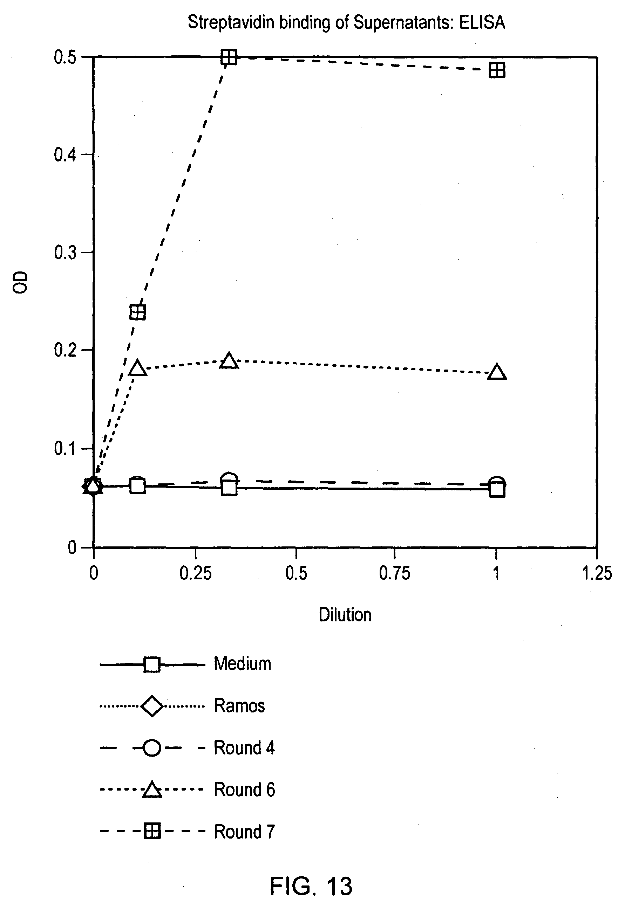

Figure 13. Streptavidin binding of IgM from the supernatants of Figure 12.

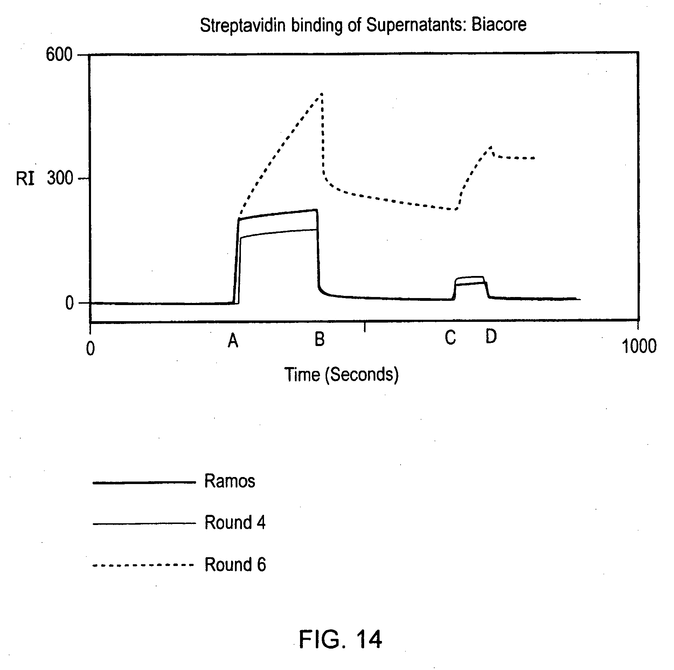

Figure 14. Streptavidin binding of supernatants from round 4 and round 6 of a selection for streptavidin binding, analysed by surface plasmon resonance.

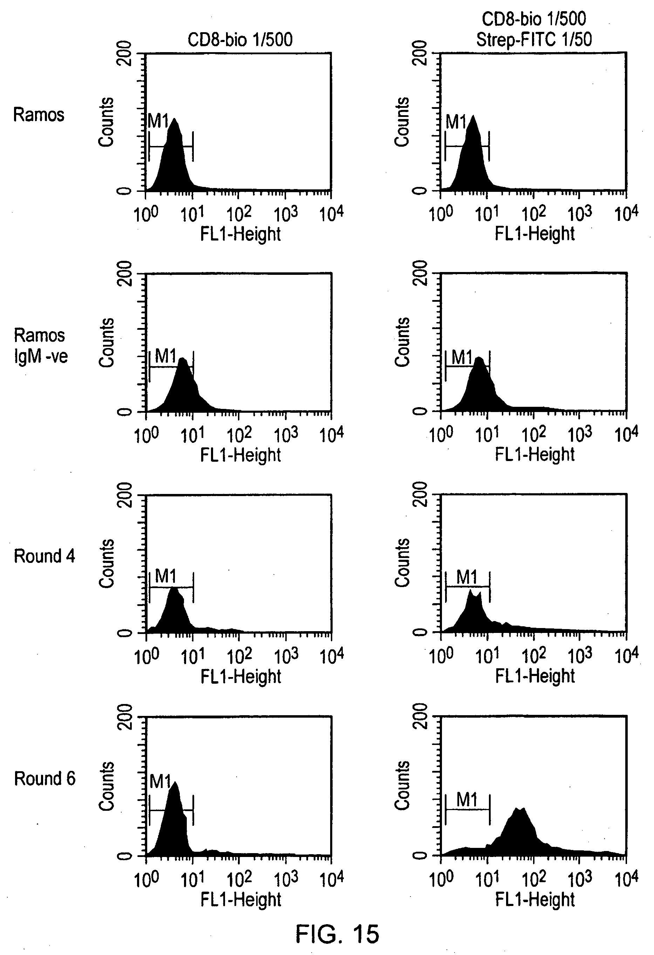

Figure 15. FACS analysis of binding to streptavidin-FITC of cells selected in rounds 4 and 6.

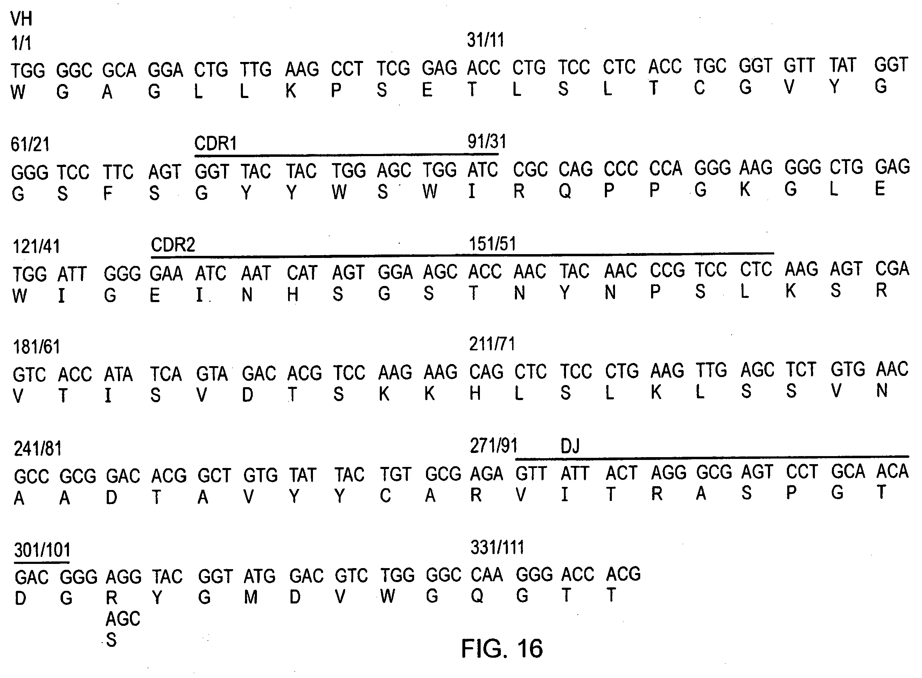

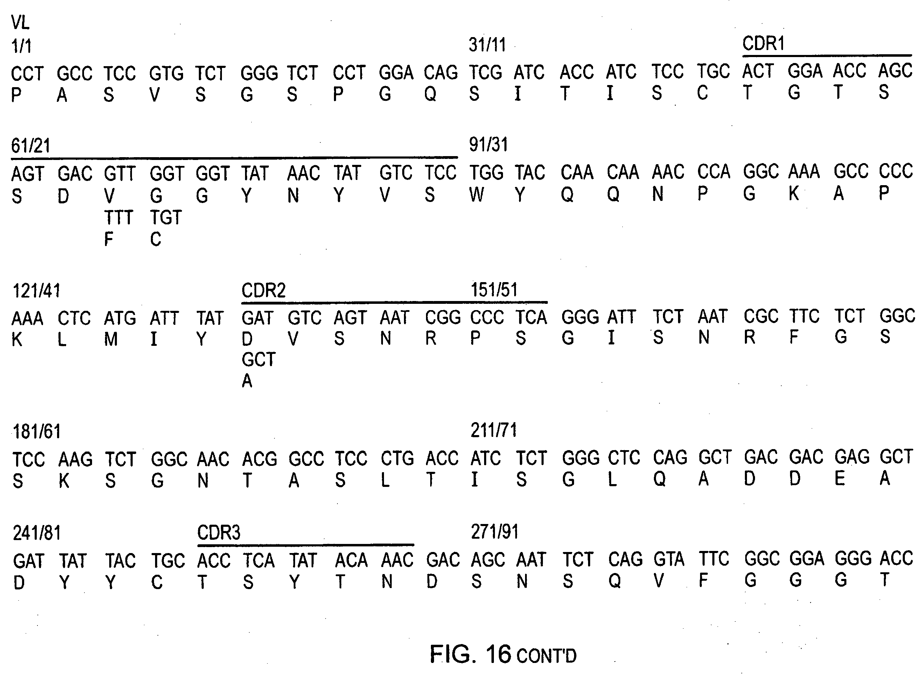

Figure 16. V_{H} and V_{L} sequences of round 6 selected IgM.

### Detailed Description of the Invention

The present invention makes available for the first time a cell line which constitutively hypermutates selected nucleic acid regions. This permits the design of systems which produce mutated gene products by a technique which mirrors affinity maturation in natural antibody production. The Ramos Burkitt line constitutively diversifies its rearranged immunoglobulin V gene during in vitro culture, This hypermutation does not require stimulation by activated T cells, exogenously-added cytokines or even maintenance of the B cell antigen receptor.

The rate of mutation (which lies in the range 0.2-1x10⁻⁴ bp⁻¹ generation⁻¹) is sufficiently high to readily allow the accumulation of a large database of unselected mutations and so reveal that hypermutation in Ramos exhibits most of the features classically associated with immunoglobulin V gene hypermutation in vivo (preferential targeting of mutation to the V; stepwise accumulation of single nucleotide substitutions; transition bias; characteristic mutational hotspots). The large majority of mutations in the unselected database are single nucleotide substitutions although deletions and duplications (sometimes with a flanking nucleotide substitution) are detectable. Such deletions and duplications have also been proposed to be generated as a consequence of hypermutation in vivo (Wilson et al., 1998; Goosens et al., 1998; Wu & Kaartinen, 1995).

The isolation of cells which constitutively hypermutate selected nucleic acid regions is based on the monitoring of V gene mutation in cell lines derived from antibody-producing cells such as B cells. The selection method employed in the disclosure may be configured in a number of ways.

### Selection of Hypermutating Cells

Hypermutating cells may be selected from a population of cells by a variety of techniques, including sequencing of target sequences, selection for expression loss mutants, assay using bacterial MutS protein and selection for change in gene product activity,

One of the features of hypermutation of target nucleic acids is that the process results in the introduction of stop codons into the target sequence with far greater frequency than would be observed in the absence of hypermutation. This results in loss of production of a gene product from the cell. This loss may be exploited to identify cells which are hypermutating nucleic acid sequences.

In a preferred embodiment of the invention, the target nucleic acid encodes an immunoglobulin. Immunoglobulin loss may be detected both for cells which secrete immunoglobulins into the culture medium, and for cells in which the immunoglobulin is displayed on the cell surface. Where the immunoglobulin is present on the cell surface, its absence may be identified for individual cells, for example by FACS analysis, immunofluorescence microscopy or ligand immobilisation to a support. In a preferred embodiment, cells may be mixed with antigen-coated magnetic beads which, when sedimented, will remove from the cell suspension all cells having an immunoglobulin of the desired specificity displayed on the surface.

The technique may be extended to any immunoglobulin molecule, including antibodies, T-cell receptors and the like. The selection of imnlunoglobulin molecules will depend on the nature of the clonal population of cells which it is desired to assay according to the invention.

Alternatively, cells according to the disclosure may be selected by sequencing of target nucleic acids, such as V genes, and detection of mutations by sequence comparison. This process may be automated in order to increase throughput.

In a further embodiment, cells which hypermutate V genes may be detected by assessing change in antigen binding activity in the immunoglobulins produced in a clonal cell population. For example, the quantity of antigen bound by a specific unit amount of cell medium or extract may be assessed in order to determine the proportion of immunoglobulin produced by the cell which retains a specified binding activity. As the V genes are mutated, so binding activity will be varied and the proportion of produced immunoglobulin which binds a specified antigen will be reduced.

Alternatively, cells may be assessed in a similar manner for the ability to develop a novel binding affinity, such as by exposing them to an antigen or mixture of antigens which are initially not bound and observing whether a binding affinity develops as the result of hypermutation.

In a further embodiment, the bacterial MutS assay may be used to detect sequence variation in target nucleic acids. The MutS protein binds to mismatches in nucleic acid hybrids. By creating heteroduplexes between parental nucleic acids and those of potentially mutated progeny, the extent of mismatch formation, and thus the extent of nucleic acid mutation, can be assessed.

Where the target nucleic acid encodes an gene product other than an immunoglobulin, selection may be performed by screening for loss or alteration of a function other than binding. For example, the loss or alteration of an enzymatic activity may be screened for.

Cells which target sequence hypermutation are assessed for mutation in other nucleic acid regions, A convenient region to assay is the constant (C) region of an immunoglobulin gene. C regions are not subject to directed hypermutation according to the invention. The assessment of C regions is preferably made by sequencing and comparison, since this is the most certain method for determining the *absence* of mutations. However, other techniques may be employed, such as monitoring for the retention of C region activities, for example complement fixation, which may be disrupted by hypermutation events.

### Adaptation of the endogenous gene products

Having obtained a cell line which constitutively hypermutates an endogenous gene, such as an immunoglobulin V region gene, the present invention provides for the adaptation of the endogenous gene product, by constitutive hypermutation, to produce a gene product having novel properties. For example, the present invention provides for the production of an immunoglobulin having a novel binding specificity or an altered binding affinity.

The process of hypermutation is employed, in nature, to generate improved or novel binding specificities in immunoglobulin molecules. Thus, by selecting cells according to the invention which produce immunoglobulins capable of binding to the desired antigen and then propagating these cells in order to allow the generation of further mutants, cells which express immunoglobulins having improved binding to the desired antigen may be isolated.

A variety of selection procedures may be applied for the isolation of mutants having a desired specificity. These include Fluorescence Activated Cell Sorting (FACS), cell separation using magnetic particles, antigen chromatography methods and other cell separation techniques such as use of polystyrene beads.

Separating cells using magnetic capture may be accomplished by conjugating the antigen of interest to magnetic particles or beads. For example, the antigen may be conjugated to superparamagnetic iron-dextran particles or beads as supplied by Miltenyi Biotec GmbH. These conjugated particles or beads are then mixed with a cell population which may express a diversity of surface immunoglobulins. If a particular cell expresses an immunoglobulin capable of binding the antigen, it will become complexed with the magnetic beads by virtue of this interaction. A magnetic field is then applied to the suspension which immobilises the magnetic particles, and retains any cells which are associated with them via the covalently linked antigen. Unbound cells which do not become linked to the beads are then washed away, leaving a population of cells which is isolated purely on its ability to bind the antigen of interest- Reagents and kits are available from various sources for performing such one-step isolations, and include Dynal Beads (Dynal AS; http://www.dynal.no), MACS-Magnetic Cell Sorting (Miltenyi Biotec GmbH; http://www.miltenyibiotec.com), CliniMACS (AmCell; http://www.amcell.com) as well as Biomag, Amerlex-M beads and others.

Fluorescence Activated Cell Sorting (FACS) can be used to isolate cells on the basis of their differing surface molecules, for example surface displayed immunoglobulins. Cells in the sample or population to be sorted are stained with specific fluorescent reagents which bind to the cell surface molecules These reagents would be the antigen(s) of interest linked (either directly or indirectly) to fluorescent markers such as fluorescein, Texas Red, malachite green, green fluorescent protein (GFP), or any other fluorophore known to those skilled in the art. The cell population is then introduced into the vibrating flow chamber of the FACS machine. The cell stream passing out of the chamber is encased in a sheath of buffer fluid such as PBS (Phosphate Buffered Saline). The stream is illuminated by laser light and each cell is measured for fluorescence, indicating binding of the fluorescent labelled antigen. The vibration in the cell stream causes it to break up into droplets, which carry a small electrical charge. These droplets can be steered by electric deflection plates under computer control to collect different cell populations according to their affinity for the fluorescent labelled antigen. In this manner, cell populations which exhibit different affinities for the antigen(s) of interest can be easily separated from those cells which do not bind the antigen. FACS machines and reagents for use in FACS are widely available from sources world-wide such as Becton-Dickinson, or from service providers such as Arizona Research Laboratories (http://www.arl.arizona.edu/facs/).

Another method which can be used to separate populations of cells according to the affinity of their cell surface protein(s) for a particular antigen is affinity chromatography. In this method, a suitable resin (for example CL-600 Sepharose, Pharmacia Inc.) is covalently linked to the appropriate antigen. This resin is packed into a column, and the mixed population of cells is passed over the column. After a suitable period of incubation (for example 20 minutes), unbound cells are washed away using (for example) PBS buffer. This leaves only that subset of cells expressing immunoglobulins which bound the antigen(s) of interest, and these cells are then eluted from the column using (for example) an excess of the antigen of interest, or by enzymatically or chemically cleaving the antigen from the resin. This may be done using a specific protease such as factor X, thrombin, or other specific protease known to those skilled in the art to cleave the antigen from the column via an appropriate cleavage site which has previously been incorporated into the antigen-resin complex. Alternatively, a non-specific protease, for example trypsin, may be employed to remove the antigen from the resin, thereby releasing that population of cells which exhibited affinity for the antigen of interest.

### Insertion of heterologous transcription units

In order to maximise the chances of quickly selecting an antibody variant capable of binding to any given antigen, or to exploit the hypermutation system for non-immunoglobulin genes, a number of techniques may be employed to engineer cells according to the invention such that their hypermutating abilities may be exploited.

In a first embodiment, transgenes are transfected into a cell according to the invention such that the transgenes become targets for the directed hypermutation events,

As used herein, a "transgene" is a nucleic acid molecule which is inserted into a cell, such as by transfection or transduction. For example, a "transgene" may comprise a heterologous transcription unit as referred to above, which may be inserted into the genome of a cell at a desired location.

The plasmids used for delivering the transgene to the cells are of conventional construction and comprise a coding sequence, encoding the desired gene product, under the control of a promoter. Gene transcription from vectors in cells according to the invention may be controlled by promoters derived from the genomes of viruses such as polyoma virus, adenovirus, fowlpox virus, bovine papilloma virus, avian sarcoma virus, cytomegalovirus (CMV), a retrovirus and Simian Virus 40 (SV40), from heterologous mammalian promoters such as the actin promoter or a very strong promoter, e.g. a ribosomal protein promoter, and from the promoter normally associated with the heterologous coding sequence, provided such promoters are compatible with the host system of the invention.

Transcription of a heterologous coding sequence by cells according to the invention may be increased by inserting an enhancer sequence into the vector Enhancers are relatively orientation and position independent. Many enhancer sequences are known from mammalian genes (e.g. elastase and globin). However, typically one will employ an enhancer from a eukaryotic cell virus. Examples include the SV40 enhancer on the late side of the replication origin (bp 100-270) and the CMV early promoter enhancer. The enhancer may be spliced into the vector at a position 5' or 3' to the coding sequence, but is preferably located at a site 5' from the promoter.

Advantageously, a eukaryotic expression vector may comprise a locus control region (LCR), LCRs are capable of directing high-level integration site independent expression of transgenes integrated into host cell chromatin, which is of importance especially where the heterologous coding sequence is to be expressed in the context of a permanently-transfected eukaryotic cell line in which chromosomal integration of the vector has occurred, in vectors designed for gene therapy applications or in transgenic animals.

Eukaryotic expression vectors will also contain sequences necessary for the termination of transcription and for stabilising the mRNA. Such sequences are commonly available from the 5' and 3' untranslated regions of eukaryotic or viral DNAs or cDNAs. These regions contain nucleotide segments transcribed as polyadenylated fragments in the untranslated portion of the mRNA.

An expression vector includes any vector capable of expressing a coding sequence encoding a desired gene product that is operatively linked with regulatory sequences, such as promoter regions, that are capable of expression of such DNAs. Thus, an expression vector refers to a recombinant DNA or RNA construct, such as a plasmid, a phage, recombinant virus or other vector, that upon introduction into an appropriate host cell, results in expression of the cloned DNA. Appropriate expression vectors are well known to those with ordinary skill in the art and include those that are replicable in eukaryotic and/or prokaryotic cells and those that remain episomal or those which integrate into the host cell genome. For example, DNAs encoding a heterologous coding sequence may be inserted into a vector suitable for expression of cDNAs in mammalian cells, e.g. a CMV enhancer-based vector such as pEVRF (Matthias, et al., 1989),

Construction of vectors according to the invention employs conventional ligation techniques. Isolated plasmids or DNA fragments are cleaved, tailored, and religated in the form desired to generate the plasmids required. If desired, analysis to confirm correct sequences in the constructed plasmids is performed in a known fashion. Suitable methods for constructing expression vectors, preparing in vitro transcripts, introducing DNA into host cells, and performing analyses for assessing gene product expression and function are known to those skilled in the art. Gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe which may be based on a sequence provided herein. Those skilled in the art will readily envisage how these methods may be modified, if desired.

In one variation of the first embodiment, transgenes according to the invention also comprise sequences which direct hypermutation. Such sequences have been characterised, and include those sequences set forth in Klix *et al.,* (1998), and Sharpe *et al.,* (1991). Thus, an entire locus capable of expressing a gene product and directing hypermutation to the transcription unit encoding the gene product is transferred into the cells. The transcription unit and the sequences which direct hypermutation are thus exogenous to the cell However, although exogenous the sequences which direct hypermutation themselves may be similar or identical to the sequences which direct hypermutation naturally found in the cell

In a second embodiment, the endogenous V gene(s) or segments thereof may be replaced with heterologous V gene(s) by homologous recombination, or by gene targeting using, for example, a Lox/Cre system or an analogous technology or by insertion into hypermutatting cell lines which have spontaneously deleted endogenous V genes. Alternatively, V region gene(s) may be replaced by exploiting the observation that hypermutation is accompanied by double stranded breaks in the vicinity of rearranged V genes.

The invention is further described below, for the purposes of illustration only, in the following examples.

### Example 1: Selection of a hypermutating cell

In order to screen for a cell that undergoes hypermutation in vitro, the extent of diversity that accumulates in several human Burkitt lymphomas during clonal expansion is assessed. The Burkitt lines BL2, BL41 and BL70 are kindly provided by G. Lenoir (IARC, Lyon, France) and Ramos (Klein et al., 1975) is provided by D. Fearon (Cambridge, UK). Their rearranged V_{H} genes are PCR amplified from genomic DNA using multiple V_{H} family primers together with a J_{H} consensus oligonucleotide. Amplification of rearranged V_{H} segments is accomplished using Pfu polymerase together with one of 14 primers designed for each of the major human V_{H} families (Tomlinson, 1997) and a consensus J_{H} back primer which anneals to all six human J_{H} segments (JOL48, 5'-GCGGTACCTGAGGAGACGGTGACC-3', gift of C. Jolly). Amplification of the Ramos V_{H} from genomic DNA is performed with oligonucleotides RVHFOR (5'-CCCCAAGCTTCCCAGGTGCAGCTACAGCAG) and JOL48, Amplification of the expressed V_{H}-C_{µ} cDNA is performed using RVHFOR and Cµ2BACK (5'-CCCCGGTACCAGATGAGCTTGGACTTGCGG). The genomic Cµ1/2 region is amplified using Cµ2BACK with Cµ1FOR (5'-CCCCAAGCTTCGGGAGTGCATCCGCCCCAACCCTT); the functional Cµ allele of Ramos contains a C at nucleotide 8 of Cµ2 as opposed to T on the non-functional allele. Rearranged V_{λ}s are amplified using 5'-CCCCAAGCTTCCCAGTCTGCCCTGACTCAG and 5'-CCCCTCTAGACCACCTAGGACGGTC-AGCTT. PCR products are purified using QIAquick (Qiagen) spin columns and sequenced using an ABI377 sequencer following cloning into M13. Mutations are computed using the GAP4 alignment program (Bonfield et al., 1995).

Sequencing of the cloned PCR products reveals considerable diversity in the Ramos cell line (a prevalence of 2.8x10⁻³ mutations bp⁻¹ in the V_{H}) although significant heterogeneity is also observed in BL41 as well as in BL2. See Figure 1A. Sequence diversity in the rearranged V_{H} genes of four sporadic Burkitt lymphoma lines are shown as pie charts. The rearranged V_{H} genes in each cell line are PCR amplified and cloned into M13. For each cell line, the consensus is taken as the sequence common to the greatest number of M13 clones and a germline counterpart (indicated above each pie) assigned on the basis of closest match using the VBASE database of human immunoglobulin sequences (Tomlinson, 1997). The V_{H} consensus sequence for Ramos used herein differs in 3 positions from the sequence determined by Chapman et al (1996), five positions from that determined by Ratech (1992) and six positions from its closest germline counterpart V_{H}4(DP-63).

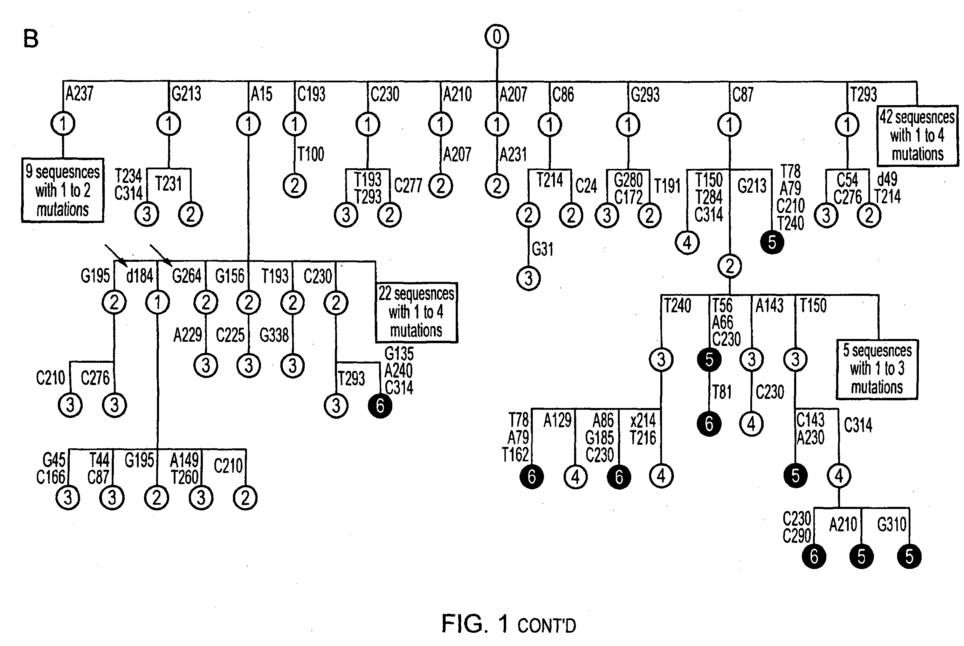

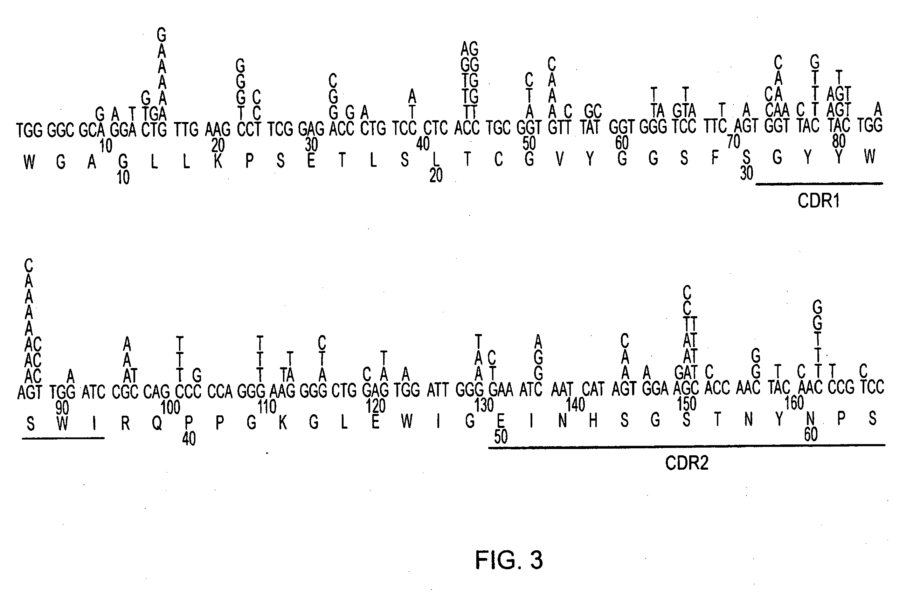

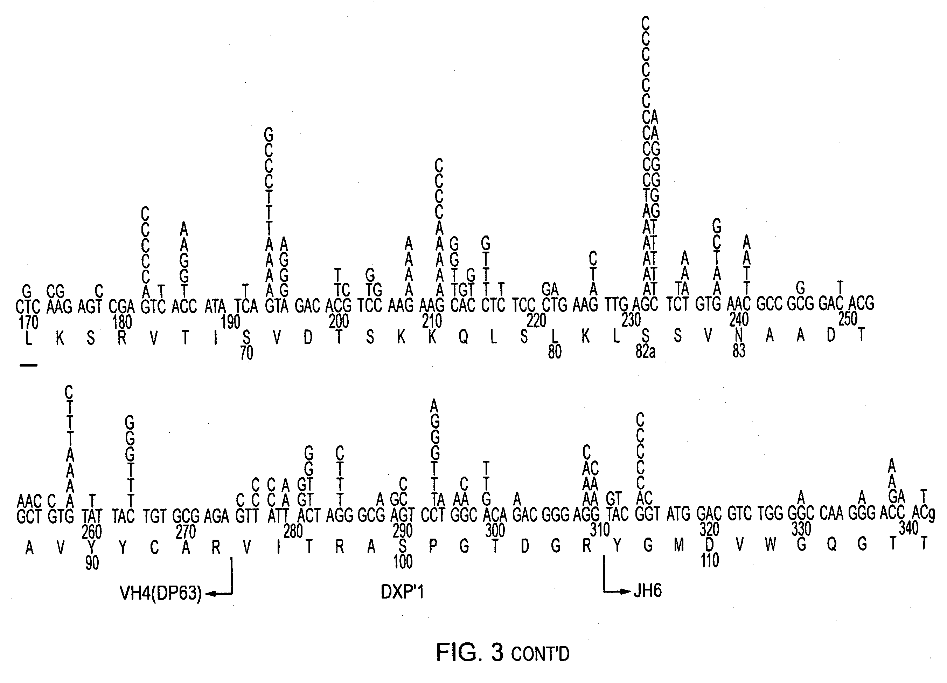

The analysis of V_{H} diversity in Ramos is extended by sequencing the products from nine independent PCR amplifications. This enables a likely dynastic relationship between the mutated clones in the population to be deduced, minimising the number of presumed independent repeats of individual nucleotide substitutions (Figure 1B). 315 M13V_{H} clones obtained from nine independent PCR amplifications are sequenced; the dynasty only includes sequences identified (rather than presumed intermediates). Individual mutations are designated according to the format "C230" with 230 being the nucleotide position in the Ramos V_{H} (numbered as in Figure 3) and the "C" indicating the novel base at that position. The criterion used to deduce the genealogy is a minimisation of the number of independent occurrences of the same nucleotide substitution. The majority of branches contain individual members contributed by distinct PCR amplifications The rare deletions and duplications are indicated by the prefix "x" and "d" respectively. Arrows highlight two mutations (a substitution at position 264 yielding a stop codon and a duplication at position 184) whose position within the tree implies that mutations can continue to accumulate following loss of functional heavy chain expression.

PCR artefacts make little contribution to the database of mutations; not only is the prevalence of nucleotide substitutions greatly in excess of that observed in control PCR amplifications (<0.05x10⁻³ bp⁻¹) but also identically mutated clones (as well as dynastically related ones) are found in independent amplifications. In many cases, generations within a lineage differ by a single nucleotide substitution indicating that only a small number of substitutions have been introduced in each round of mutation.

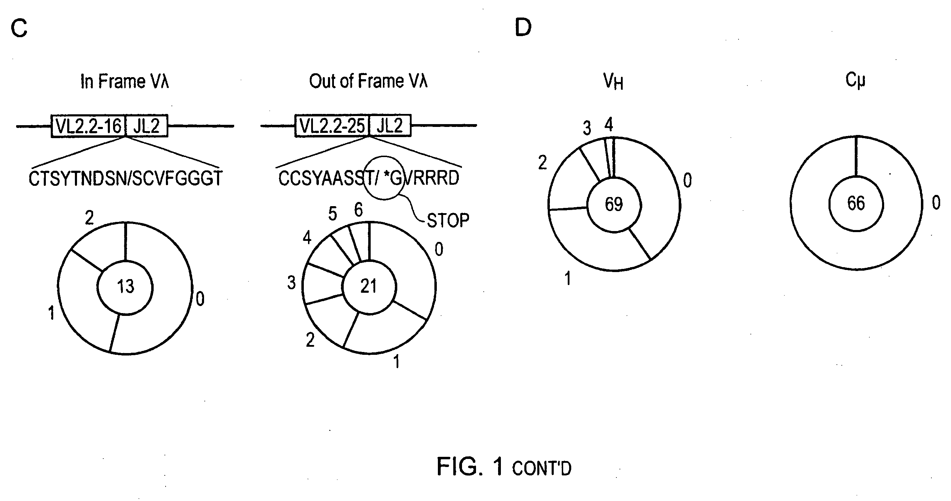

Analysis of V_{λ} rearrangements reveals that Ramos harbours an in-frame rearrangement of V_{λ}2.2-16 (as described by Chapman et al. 1996)) and an out-of-frame rearrangement of V_{λ}2.2-25. There is mutational diversity in both rearranged V_{λ}s although greater diversity has accumulated on the non-functional allele (Figure 1C).

A classic feature of antibody hypermutation is that mutations largely accumulate in the V region but scarcely in the C. This is also evident in the mutations that have accumulated in the Ramos IgH locus (Figure 1D). M13 clones containing cDNA inserts extending through V_{H}, Cµ1 and the first 87 nucleotides Cµ2 are generated by PCR from the initial Ramos culture. The Pie charts (presented as in Figure 1A) depict the extent of mutation identified in the 341 nucleotide stretch of V_{H} as compared to a 380 nucleotide stretch of Cµ extending from the beginning of Cµ1.

The IgM immunoglobulin produced by Ramos is present both on the surface of the cells and, in secreted form, in the culture medium. Analysis of the culture medium reveals that Ramos secretes immunoglobulin molecules to a very high concentration, approximately 1µg/ml. Thus, Ramos is capable of secreting immunoglobulins to a level which renders it unnecessary to redone immunoglobulin genes into expression cell lines or bacteria for production.

### Example 2: V_{H} diversification in Ramos is constitutive

To address whether V gene diversification is ongoing, the cells are cloned and V_{H} diversity assessed using a MutS-based assay after periods of *in vitro* culture. The Ramos V_{H} is PCR amplified and purified as described above using oligonucleotides containing a biotinylated base at the 5'-end. Following denaturation/renaturation (99°C for 3 min; 75 °C for 90 min), the extent of mutation is assessed by monitoring the binding of the mismatched heteroduplexed material to the bacterial mismatch-repair protein MutS, filter-bound, with detection by ECL as previously described (Jolly et al., 1997).

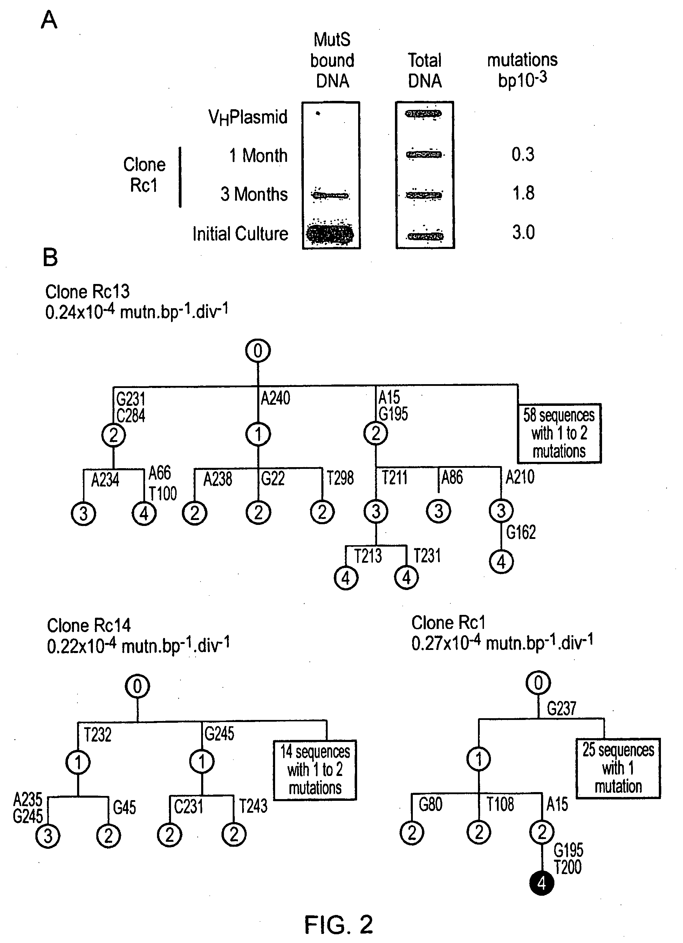

The results indicate that V_{H} diversification is indeed ongoing (see Figure 2A). DNA is extracted from Ramos cells that have been cultured for 1 or 3 months following limit dilution cloning. The rearranged V_{H} is PCR amplified using biotinylated oligonucleotides prior to undergoing denaturation/renaturation; mismatched heteroduplexes are then detected by binding to immobilised MutS as previously described (Jolly et al., 1997). An aliquot of the renatured DNA is bound directly onto membranes to confirm matched DNA loading (Total DNA control). Assays performed on the Ramos V_{H} amplified from a bacterial plasmid template as well as from the initial Ramos culture are included for comparison.

The V_{H} genes are PCR amplified from Ramos cultures that have been expanded for four (Rc1) or six (Rc13 and 14) weeks (Figure 2B). A mutation rate for each clone is indicated and is calculated by dividing the prevalence of independent V_{H} mutations at 4 or 6 weeks post-cloning by the presumed number of cell divisions based on a generation time of 24 h. The sequences reveal step-wise mutation accumulation with a mutation rate of about 0.24x10⁻⁴ mutations bp⁻¹ generations⁻¹.

Direct comparison of the V_{H} mutation rate in Ramos to that in other cell-lines is not straightforward since there is little information on mutation rates in other lines as judged by unselected mutations incorporated throughout the V_{H} obtained following clonal expansion from a single precursor cell. However, the prevalence of mutations following a two week expansion of 50 precursor BL2 cells has been determined under conditions of mutation induction (2.7x10⁻³ mutations bp⁻¹; Denépoux et al , 1997). Similar experiments performed with Ramos under conditions of normal culture reveal a mutation prevalence of 2.3x10⁻³ mutations bp⁻¹. Various attempts to enhance the mutation rate by provision of cytokines, helper T cells etc. have proved unsuccessful. Thus, the rate of mutation that can be achieved by specific induction in BL2 cells appears to be similar to the constitutive rate of V_{H} mutation in Ramos.

### Example 3: Examination of the nature of V_{H} mutations in Ramos

A database of mutational events is created which combines those detected in the initial Ramos culture (from 141 distinct sequences) with those detected in four subclones that have been cultured in various experiments without specific selection (from a further 135 distinct sequences). This database is created after the individual sets of sequences have been assembled into dynastic relationships (as detailed in the legend to Figure 1B) to ensure that clonal expansion of an individual mutated cell does not lead to a specific mutational event being counted multiple times. Here an analysis of this composite database of 340 distinct and presumably unselected mutational events (200 contributed by the initial Ramos culture and 140 from the expanded subclones) is described; separate analysis of the initial and subclone populations yields identical conclusions.

The overwhelming majority of the mutations (333 out of 340) are single nucleotide substitutions. A small number of deletions (4) and duplications (3) are observed but no untemplated insertions; these events are further discussed below. There are only five sequences which exhibited nucleotide substitutions in adjacent positions; however, in three of these five cases, the genealogy revealed that the adjacent substitutions have been sequentially incorporated. Thus, the simultaneous creation of nucleotide substitutions in adjacent positions is a rare event.

The distribution of the mutations along the V_{H} is highly non-random (See Figure 3). Independently occurring base substitutions are indicated at each nucleotide position. The locations of CDR1 and 2 are indicated. Nucleotide positions are numbered from the 3'-end of the sequencing primer with nucleotide position +1 corresponding to the first base of codon 7; codons are numbered according to Kabat. Mutations indicated in italics (nucleotide position 15, 193, 195 and 237) are substitutions that occur in a mutated subclone and have reverted the sequence at that position to the indicated consensus.

The major hotspot is at the G and C nucleotides of the Ser82a codon, which has previously been identified as a major intrinsic mutational hotspot in other V_{H} genes (Wagner et al., 1995; Jolly et al., 1996) and conforms to the RGYW consensus (Rogozin and Kolchanov, 1992; Betz et al., 1993). Whilst the dominant intrinsic mutational hotspot in many V_{H} genes is at Ser31, this codon is not present in the Ramos consensus V_{H} (or its germline counterpart) which have Gly at that position, The individual nucleotide substitutions show a marked bias in favour of transitions (51% rather than randomly-expected 33%). There is also a striking preference for targeting G and C which account for 82% of the nucleotides targeted (Table 1).

**Table 1. Nucleotide substitution preferences of hypermutation in Ramos**

| | Frequency of substitution to | | | | |
|---|---|---|---|---|---|
| | T | C | G | A | Total |
| Parental nucleotide | | | | | |
| T | - | 3.9 | 1.2 | 3.0 | 8.1 |
| C | 17.4 | - | 12.6 | 4.8 | 34.8 |
| G | 7.2 | 15.9 | - | 24.0 | 47.1 |
| A | 2.4 | 1.8 | 5.7 | - | 9.9 |
| Single nucleotide substitutions were computed on the V_{H} coding strand and are given as the percentage of the total number (333) of independent, unselected nucleotide substitutions identified. | | | | | |

### Example 4: Selection of hypermutating cells by IgM-loss

Analysis of the Ramos variants reveals several mutations that must have inactivated V_{H} (see Figure 1B) suggesting it might be possible for the cells to lose IgM expression but remain viable. If this is the case, Ig expression loss would be an easy means to select a constitutively hypermutating B cell line.

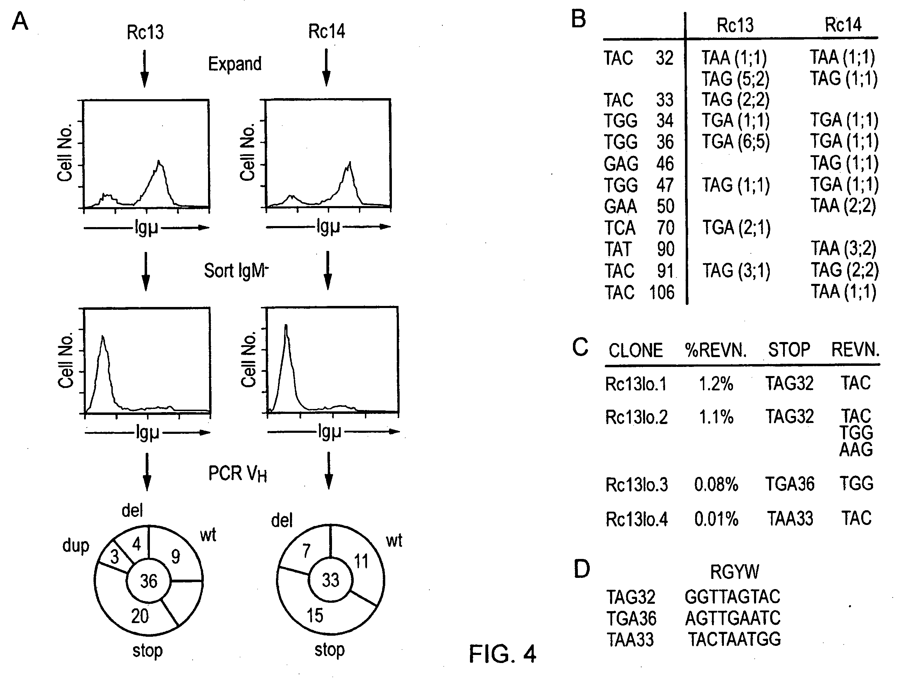

Analysis of the Ramos culture reveals it to contain 8% surface IgM- cells. Such IgM-loss variants are generated during in vitro culture, as follows. The starting Ramos culture is transfected with a pSV2neo plasmid, diluted into 96-well plates and clones growing in selective medium allowed to expand. Flow cytometry performed on the expanded clones six months after the original transfection reveals the presence of IgM-loss variants, constituting 16% and 18% of the two clonal populations (Rc13 and Rc14) shown here (Figure 4A). Enrichment by a single round of sorting yields subpopulations that contain 87% (Rc13) and 76% (Rc14) surface IgM-negative cells. Following PCR amplification of the rearranged V_{H} gene in these subpopulations, sequencing reveals that 75% (Rc13) and 67% (Rc14) of the cloned V_{H} segments contained a nonsense (stop), deletion (del) or duplication (dup) mutation within the 341 nucleotide V_{H} stretch analysed. The remainder of the clones are designated wild type (wt) although no attempt is made to discriminate possible V_{H}-inactivating missense mutations. The 4 deletions and 3 duplications identified in the Rc13 population are all distinct whereas only 4 distinct mutations account for the 7 Rc14 sequences determined that harbour deletions. The nature of the deletions and duplications is presented in Figure 6: each event is named with a letter followed by a number. The letter gives the provenance of the mutation (A, B and C being the cloned TdT⁻ control transfectants, D, E and F the TdT⁺ transfectants and U signifies events identified in the initial, unselected Ramos culture); the number indicates the first nucleotide position in the sequence string. Nucleotides deleted are specified above the line and nucleotides added (duplications or non-templated insertions) below the line; single nucleotide substitutions are encircled with the novel base being specified. The duplicated segments of V_{H} origin are underlined; non-templated insertions are in bold. With several deletions or duplications, the event is flanked by a single nucleotide of unknown provenance. Such flanking changes could well arise by nucleotide substitution (rather than non-templated insertion) and these events therefore separately grouped; the assignment of the single base substitution (encircled) to one or other end of the deletion/duplication is often arbitrary.

The IgM⁻ cells are enriched in a single round of sorting prior to PCR amplification and cloning of their V_{H} segments. The sequences reveal a considerable range of V_{H}-inactivating mutations (stop codons or frameshifts) (Figure 4) although diverse inactivating mutations are even evident in IgM-loss variants sorted after only 6 weeks of clonal expansion (see Figure 5). In Figure 5A expression of TdT in three pSV-pβG/TdT and three control transfectants of Ramos is compared by Western blot analysis of nuclear protein extracts" Nalm6 (a TdT-positive human pre-B cell lymphoma) and HMy2 (a TdT-negative mature human B lymphoma) provided controls.
In Figure 5B, pie charts are shown depicting independent mutational events giving rise to IgM-loss variants IgM⁻ variants (constituting 1-5% of the population) are obtained by sorting the three TdT⁺ and three TdT- control transfectants that have been cultured for 6 weeks following cloning. The V_{H} regions in the sorted subpopulations are PCR amplified and sequenced. The pie charts depict the types of mutation giving rise to V_{H} inactivation with the data obtained from the TdT⁺ and TdT⁻ IgM⁻ subpopulations separately pooled. Abbreviations are as in Figure 4A except that "ins" indicates clones containing apparently non-templated nucleotide insertions. Clones containing deletions or duplications together with multiple nucleotide non-templated insertions are only included within the "ins" segment of the pie. Only unambiguously distinct mutational events are computed. Thus, of the 77 distinct V_{H}-inactivating mutations identified in the TdT⁺ IgM-loss subpopulations, 30 distinct stop codon mutations are identified; if the same stop codon have been independently created within the IgM-loss population derived from a single Ramos transfectant, this would have been underscored,

The stop codons are created at variety of positions (Figure 4B) but are not randomly located. Figure 4B summarises the nature of the stop codons observed in the Rc13 and Rc14 IgM-loss populations, At least eight independent mutational events yield the nonsense mutations which account for 20 out of the 27 non-functional V_{H} sequences in the Rc13 database; a minimum of ten independent mutational events yield the nonsense mutations which account for 15 of the 22 non-functional V_{H} sequences in the Rc14 database. The numbers in parentheses after each stop codon give the number of sequences in that database that carry the relevant stop codon followed by the number of these sequences that are distinct, as discriminated on the basis of additional mutations. Analysis of stop codons in IgM-loss variants selected from four other clonal populations reveals stop codon creation at a further five locations within V_{H}. In data obtained in six independent experiments, stop codon creation is restricted to 16 of the 39 possible sites; the DNA sequences at these preferred sites being biased (on either coding or non-coding strand) towards the RGYW consensus.

Not surprisingly, whereas deletions and insertions account for only a small proportion of the mutations in unselected Ramos cultures (see above), they make a much greater contribution when attention is focused on V_{H}-inactivating mutations. It is notable that a large proportion of the IgM-loss variants can be accounted for by stop-codon/frameshift mutations in the V_{H} itself. This further supports the proposal that hypermutation in Ramos is preferentially targeted to the immunoglobulin V domain - certainly rather than the C domain or, indeed other genes (such as the Igα/Igβ sheath) whose mutation could lead to a surface IgM⁻ phenotype. It also may well be that the Ramos V_{H} is more frequently targeted for hypermutation than its productively rearranged V_{λ}, a conclusion supported by the pattern of mutations in the initial culture (Figure 1C).

Selection of cells by detection of Ig loss variants is particularly useful where those variants are capable of reverting, i.e. of reaquiring their endogenous Ig-expressing ability. The dynasty established earlier (Figure 1B) suggests not only that IgM-loss cells could arise but also that they might undergo further mutation. To confirm this, IgM-loss variants sorted from Rc13 are cloned by limiting dilution. Three weeks after cloning, the presence of IgM⁺ revertants in the IgM⁻ subclones is screened by cytoplasmic immunofluorescence analysis of 5x10⁴ cells; their prevalence is given (Figure 4C). These IgM⁺ revertants are then enriched in a single round of sorting and the V_{H} sequences of the clonal IgM⁻ variant compared to that it of its IgM⁺ revertant descendants.

Cytoplasmic immunofluorescence of ten expanded clonal populations reveals the presence of IgM⁺ revertants at varying prevalence (from 0.005% to 1.2%; Figure 4C) allowing a mutation rate of 1x10⁻⁴ mutations bp⁻¹ generation⁻¹ to be calculated by fluctuation analysis. This is somewhat greater than the rate calculated by direct analysis of unselected mutations (0.25x10⁻⁴ mutations bp⁻¹ generation⁻¹; see above), probably in part reflecting that different IgM-loss clones revert at different rates depending upon the nature of the disrupting mutation. Indeed, the sequence surrounding the stop codons in the IgM-loss derivatives of Rc13 reveals that TAG32 conforms well to the RGYW consensus (R = purine, Y = pyrimidine and W = A or T; Rogozin and Kolchanov, 1992) which accounts for a large proportion of intrinsic mutational hotspots (Betz et al., 1993) whereas TAA33 and TGA36 do not (Figure 4D).

### Example 5: Selection of a novel Ig binding activity

In experiments designed to demonstrate development of novel binding affinities, it is noted that most members of the Ramos cell line described below express a membrane IgM molecule which binds anti-idiotype antibodies (anti-Id1 and anti-Id2), specifically raised against the Ramos surface IgM. However, a few cells retain a surface IgM, yet fail to bind the anti-idiotype antibody. This is due to an alteration in binding affinity in the surface IgM molecule, such that it no longer binds antibody. Cells which express a surface IgM yet cannot bind antibody can be selected in a single round of cell sorting according to the invention.

This is demonstrated by isolating µ positive/id-negative clones which have lost the capacity to bind to anti-Id2 despite the retention of a surface IgM, by ELISA. The clones are sequenced and in six independent clones a conserved V_{H} residue, K70, is found to be mutated to N, M or R as follows:

| **Clone** | **Mutation** | |
|---|---|---|
| 2 | K70N | AAG-AAC |
| | S77N | AGC-AAC |
| 4 | K70M | AAG-ATG |
| 9 | S59R | AGT-AGG |
| | K70N | AAG-AAC |
| 10 | K70N | AAG-AAC |
| 12 | K70N | AAG-AAC |
| 13 | K70R | AAG-AGG |

No mutations were observed in the light chain. Thus, it is apparent that mutants may be selected from the Ramos cell line in which the Ig molecule produced has a single base-pair variation with respect to the parent clone.

Making use of an anti-Id1, a similar population of cells is isolated which retain expression of the Igµ constant region but which have lost binding to the anti-idiotype antibody. These cells are enriched by sorting cytometry and the sequence of V_{H} determined (Figure 7). This reveals six mutations when compared with the consensus sequence of the staring population. Two of these mutations result in amino acid sequence changes around CDR3 (R->T at 95 and P->H at 98). Thus, selection of more subtle changes in the immunoglobulin molecule are selectable by assaying for loss of binding.

In further experiments, hypermutating cells according to the invention are washed, resuspended in PBS/BSA (10⁸ cells in 0.25ml) and mixed with an equal volume of PBS/BSA containing 10% (v/v) antigen-coated magnetic beads. In the present experiment, streptavidin coated magnetic beads (Dynal) are used. After mixing at 4° C on a roller for 30 mins, the beads are washed three times with PBS/BSA, each time bringing down the beads with a magnet and removing unbound cells remaining cells are then seeded onto 96 well plates and expanded up to 10⁸ cells before undergoing a further round of selection. Multiple rounds of cell expansion (accompanied by constitutively-ongoing hypermutation) and selection are performed. After multiple rounds of selection, the proportion of cells which bind to the beads, which is initially at or close to background levels of 0.02%, begins to rise.

After 4 rounds, enrichment of streptavidin binding cells is seen. This is repeated on the fifth round (Figure 8). The low percentage recovery reflects saturation of the beads with cells since changing the cell:bead ratio from vast excess to 1:2 allows a recovery of approximately 20% from round five streptavidin binding cells (Figure 9). This demonstrates successful selection of a novel binding specificity from the hypermutating Ramos cell line, by four rounds of iterative selection.

Nucleotide sequencing of the heavy and light chains from the streptavidin binding cells predicts one amino acid change in V_{H} CDR3 and four changes in V_{L} (1 in FR1, 2 in CDR1 and 1 in CDR2) when compared with the consensus sequence of the starting population (Figure11).

To ensure that the binding of streptavidin is dependent on expression of surface immunoglobulin, immunoglobulin negative variants of the streptavidin binding cells are enriched by sorting cytometry. This markedly reduces the recovery of streptavidin binding cells with an excess of beads. The cells recovered by the Dynal-streptavidin beads from the sorted negative cells are in fact Igµ positive and most likely represent efficient recovery of Igµ streptavidin binding cells contaminating the immunoglobulin negative sorted cell population.

Preliminary data suggest that the efficiency of recovery is reduced as the concentration of streptavidin on the beads is reduced (Figure 9). This is confirmed by assaying the recovery of streptavidin binding cells with beads incubated with a range of concentrations of streptavidin (Figure 10), The percentage of cells recoverable from a binding population is dictated by the ratio of beads to cells. In this experiment the ratio is < 1: 1 beads:cells.

In a further series of experiments, a further two rounds of selection are completed, taking the total to 7. This is accomplished by reducing the concentration of streptavidin bound to the beads from 50µg/ml in round 5 to 10ug/ml in round 7. Although the secretion levels of IgM is comparable for the populations selected in rounds 4 to 7 (Figure 12), streptavidin binding as assessed by ELISA is clearly greatly increased in rounds 6 and 7, in comparison with round 4 (Figure 13).

This is confirmed by assessment of binding by Surface Plasmon Resonance on a BiaCore chip coated with streptavidin (Figure 14). The supernatant from round 7 is injected to flow across the chip at point A, and stopped at point B. At point C, antihuman IgM is injected, to demonstrate that the material bound to the streptavidin is IgM. The gradient A-B represents the association constant, and the gradient B-C to dissociation constant. From the BiaCore trace it is evident that round 6 supernatant displays superior binding characteristics to that isolated from round 4 populations or unselected Ramos cells.

Antibodies from round 6 of the selection process also show improved binding with respect to round 4. Binding of cells from round 6 selections to streptavidin-FITC aggregates, formed by preincubation of the fluorophore with a biotinylated protein, can be visualised by FACS, as shown in Figure 15. Binding to round 4 populations, unselected Ramos cells or IgM negative Ramos is not seen, indicating maturation of streptavidin binding.

Use of unaggregated streptavidin-FITC does not produce similar results, with the majority of round 6 cells not binding. This, in agreement with ELISA data, suggests that binding to streptavidin is due to avidity of the antibody binding to an array of antigen, rather than to a monovalent affinity. Higher affinity binders may be isolated by sorting for binding to non-aggregated streptavidin-FITC.

In order to determine the mutations responsible for the increased binding seen in round 6 cells over round 4 cells, the light and heavy chain antibody genes are amplified by PCR, and then sequenced. In comparison with round 4 cells, no changes in the heavy chain genes are seen, with the mutation R103S being conserved. In the light chain, mutations V23F and G24C are also conserved, but an additional mutation is present at position 46. Wild-type Ramos has an Aspartate at this position, whilst round 6 cells have an Alanine. Changes at this position are predicted to affect antigen binding, since residues in this region contribute to CDR2 of the light chain (Figure 16). It seems likely that mutation D46A is responsible for the observed increase in binding to streptavidin seen in round 6 cells,

Example 6: Construction of transgene comprising hypermutation-directing sequences

It is known that certain elements of Ig gene loci are necessary for direction of hypermutation events *in vivo.* For example, the intron enhancer and matrix attachment region Ei/MAR has been demonstrated to play a critical role (Betz *et al.,* 1994). Moreover, the 3' enhancer E3' is known to be important (Goyenechea *et al.,* 1997), However, we have shown that these elements, whilst necessary, are not sufficient to direct hypermutation in a transgene.

In contrast, provision of Ei/MAR and E3' together with additional J_{κ}-C_{κ} intron DNA and C_{κ} is sufficient to confer hypermutability. A βG-Cκ transgene is assembled by joining an 0.96 Kb PCR-generated KpnI-SpeI β-globin fragment (that extends from -104 with respect to the β-globin transcription start site to +863 and has artificial KpnI and SpeI restriction sites at its ends) to a subfragment of LκΔ[3'F1] [Betz *et al.,* 1994] that extends from nucleotide 2314 in the sequence of Max *et al* [1981] through Ei/MAR, C_{κ} and E3', and includes the 3'F1 deletion.

Hypermutation is assessed by sequencing segments of the transgene that are PCR amplified using Pfu polymerase. The amplified region extends from immediately upstream of the transcription start site to 300 nucleotides downstream of J_{κ}5.

This chimeric transgene is well targeted for mutation with nucleotide substitutions accumulating at a frequency similar to that found in a normal Igκ transgene. This transgene is the smallest so far described that efficiently recruits hypermutation and the results indicate that multiple sequences located somewhere in the region including and flanking C_{κ} combine to recruit hypermutation to the 5'-end of the β-globin/Igκ chimaera.

The recruitment of hypermutation can therefore be solely directed by sequences lying towards the 3'-end of the hypermutation domain. However, the 5'-border of the mutation domain in normal Ig genes in the vicinity of the promoter, some 100-200 nucleotides downstream of the transcription start site. This positioning of the 5'-border of the mutation domain with respect to the start site remains even in the βG-Cκ transgene when the β-globin gene provides both the promoter and the bulk of the mutation domain. These results are consistent with findings made with other transgenes indicating that it is the position of the promoter itself that defines the 5'-border of the mutation domain.

The simplest explanation for the way in which some if not all the κ regulatory elements contribute towards mutation recruitment is to propose that they work by bringing a hypermutation priming factor onto the transcription initiation complex. By analogy with the classic studies on enhancers as transcription regulatory elements, the Igκ enhancers may work as regulators of hypermutation in a position and orientation-independent manner. Indeed, the data obtained with the βG-Cκ transgene together with previous results in which E3' was moved closer to C_{κ} [Betz *et al.,* 1994] reveal that the hypermutation-enhancing activity of E3' is neither especially sensitive to its position or orientation with respect to the mutation domain.

Ei/MAR normally lies towards the 3'-end of the mutation domain. Whilst deletion of Ei/MAR drastically reduces the efficacy of mutational targeting, its restoration to a position upstream of the promoter (and therefore outside the transcribed region) gives a partial rescue of mutation but without apparently affecting the position of the 5'-border of the mutational domain. Independent confirmation of these results was obtained in transgenic mice using a second transgene, *tk-neo::*Cκ, in which a *neo* transcription unit (under control of the HSV*tk* promoter) is integrated into the C_{κ} exon by gene targeting in embryonic stem cells [Zou, *et al.,* 1995]. In this mouse, following V_{κ}-J_{κ} joining, the Igκ Ei/MAR is flanked on either side by transcription domains: the V gene upstream and *tk::neo* downstream. The *tk-neo* gene is PCR amplified from sorted germinal centre B cells of mice homozygous for the *neo* insertion.

For the *tk-neo* insert in *tk-neo::C*_{κ} mice, the amplified region extends from residues 607 to 1417 [as numbered in plasmid pMCNeo (GenBank accession U43611)], and the nucleotide sequence determined from position 629 to 1329. The mutation frequency of endogenous VJ_{κ} rearrangements in *tk-neo* ::C_{κ} mice is determined using a strategy similar to that described in Meyer *et al.,* 1996. Endogenous VJ_{κ5} rearrangements are amplified using a V_{κ} FR3 consensus forward primer (GGACTGCAGTCAGGTTCAGTGGCAGTGGG) and an oligonucleotide LκFOR [Gonzalez-Fernandez and Milstein, (1993) PNAS (USA) 90:9862-9866] that primes back from downstream of the J_{κ} cluster,

Although the level of mutation of the *tk-neo* is low and it is certainly less efficiently targeted for mutation than the 3'-flanking region of rearranged V_{κ} genes in the same cell population, it appears that - as with normal V genes - the mutation domain in the *neo* gene insert starts somewhat over 100 nucleotides downstream of the transcription start site despite the fact that Ei/MAR is upstream of the promoter.

Thus, transgenes capable of directing hypermutation in a constitutively hypermutating cell line may be constructed using Ei/MAR, E3' and regulatory elements as defined herein found downstream of J_{κ} Moreover, transgenes may be constructed by replacement of or insertion into endogenous V genes, as in the case of the *tk-neo* ::C_{κ} mice, or by linkage of a desired coding sequence to the J_{κ} intron, as in the case of the βG-Cκ transgene.

### References

Adetugbo, K, Milstein, C, and Secher, D.S (1977). Molecular analysis of spontaneous somatic mutants. Nature 265, 299-304.
Bahler, D.W. and Levy, R. (1992). Clonal evolution of a follicular lymphoma: Evidence for antigen selection. Proc, Natl. Acad. Sci. USA 89, 6770-6774.
Bass, S., R. Greene, and J.A. Wells. (1990). Hormone Phage: An Enrichment Method for Variant Proteins With Altered Binding Properties. Proteins. 8, 309-314.
Braeuninger, A., Küppers, R., Strickler, J.G-, Wacker, H.-H.., Rajewsky, K. and Hansmann, M.-L. (1997). Hodgkin and Reed-Sternberg cells in lymphocyte predominant disease represent clonal populations of germinal center-derived tumor B cells. Proc. Natl. Acad. Sci. USA 94, 9337-9342.
Berek, C. and Milstein, C. (1988). The dynamic nature of the antibody repertoire. Immunol. Rev. 105, 5-26.
Betz, A.G., Neuberger, M.S. and Milstein, C. (1993). Discriminating intrinsic and antigen-selected mutational hotspots in immunoglobulin V genes. Immunol. Today 14, 405-411.
Betz, A. G., Milstein, C., Gonzalez-Femandez, A., Pannell, R., Larson, T. and Neuberger, M. S., Cell 1994. 77: 239-248.
Bonfield, J.K., Smith, K.F. and Staden, R. (1995). A new DNA-sequence assembly program. Nucleic Acids Res. 23, 4992-99.
Bruggemann, M., Radbruch, A. and Rajewsky, K. (1982). Immunoglobulin V region variants in hybridoma cells. I, Isolation of a variant with altered idiotypic and antigen binding specificity. EMBO J. 1, 629-634.
Capizzi and Jameson, (1973) Mutat. Res. 17:147-8
Chapman, C.J., Mockridge, C.I., Rowe, M., Rickinson, A.B. and Stevenson, F.K. (1995), Analysis of VH genes used by neoplastic B cells in endemic Burkitt's lymphoma shows somatic hypermutation and intraclonal heterogeneity. Blood 85, 2176-2181.
Chapman, C.J, Zhou, J.X., Gregory, C., Rickinson, A.B. and Stevenson F.K. (1996). VH and VL gene analysis in sporadic Burkitt's lymphoma shows somatic hypermutation, intraclonal heterogeneity and a role for antigen selection. Blood 88, 3562-3568.
Chapman, K.B. and Szostak, J.W. (1994) Curr. op. Struct. Biol., 4, 618-622. Chui, Y.-L., Lozano, F., Jarvis, J.M., Pannell, R., and Milstein, C. (1995). A reporter gene to analyse the hypermutation of immunoglobulin genes. J. Mol. Biol. 249, 555-563.
Clackson, T and Wells, J A. (1994) Trends Biotechnol, 12, 173-84. Coffino, P. and Scharff, M.D. (1971). Rate of somatic mutation in immunoglobulin, production by mouse myeloma cells. Proc. Natl. Acad. Sci. USA 68, 219-223.
Cull, M.G., Miller, J.F. and Schatz, P.J. (1992) Proc Natl Acad Sci USA, 89, 1865-9.
Denépoux, S., Razanajaona, D., Blanchard, D., Meffre, G., Capra, J.D., Banchereau, J and Lebecque, S. (1997). Induction of somatic mutation in a human B cell line in vitro. Immunity 6, 35-46.
Drake, J.W. (1998) Genetics 148:1667-1686.
Ellington, A.D. and Szostak, J.W. (1990) Nature, 346, 81822
Gold, L., Polisky, B , Uhlenbeck, 0. and Yarus, M. (1995) Annu Rev Biochem, 64, 763-97.
Goossens, T., Klein, U. and Kiippers, R. (1998). Frequent occurrence of deletions and duplications during somatic hypermutation: Implications for oncogenic translocations and heavy chain disease. Proc. Natl. Acad. Sci. USA 95, 2463-2468
Goyenechea, B., Klix, N., Yélamos, J., Williams, G. T., Riddell, A., Neuberger, M. S. and Milstein, C., EMBO J. 1997. 16, in the press.
Green, N.S., Lin, M.M. and Scharff, M.D. (1998). Immunoglobulin hypermutation in cultured cells. Immunol. Rev. 162, 77-87.
Green, R. and Szostak, J.W. (1992) Science, 258, 1910-5.
Jain, R., Roncella, S., Hashimoto, S., Carbone, A., Franco di Celle, P., Foa, R., Ferrarini, M and Chiorazzi, N. (1994). A potential role for antigen selection in the clonal evolution of Burkitt's lymphoma. J. Immunol. 153, 45-52.
Jolly, C.J., Klix, N. and Neuberger, M.S. (1997). Rapid methods for the analysis of immunoglobulin gene hypermutation: application to transgenic and gene targeted mice. Nucleic Acids Res. 25, 1913-1919.
Jolly, C.J., Wagner, S.D., Rada, C.A., Klix, N., Milstein, C. and Neuberger, M.S. (1996). The targeting of somatic hypermutation. Semin. Immunol. 8, 159-168.
Joyce, G.F. (1994) Curr. op. Structural Biol., 4, 331-336.
Klein, G., Giovanella, B., Westman, A., Stehlin, J. and Mumford, D. (1975). An EBV negative cell line established from a American Burkitt lymphoma; receptor characteristics, EBV infectability and permanent conversion into EBV-positive sublines by in vitro infection, Intervirology 5, 319-334,
Klix et al., (1998) Eur. J. Immunol. 28:317-326.
Lundberg, K.S., et al., (1991) Gene 108:1-6.
Luria and Delbreck., (1943) Genetics 28:491-511
McCafferty, J., Griffiths, A.D., Winter, G. and Chiswell, D.J. (1990) Nature, 348, 552-4.
McKean, D., Huppi, K., Bell, M., Staudt, L., Gerhard, W. and Weigert, M. (1984).
Generation of antibody diversity in the immune response of BAL.B/c mice to influenza virus haemagglutinin. Proc. Natl, Acad. Sci. USA 81, 3180-3184.
Mattheakis, L.C., Bhatt, R.R. and Dower, W,J. (1994) Proc Natl Acad Sci USA, 91, 9022-6.
Matthias, et al. , (1989) NAR 17, 6418.
Max, E. E., Maizel, J. V. and Leder, P., J. Biol. Chem. 1981. 256: 5116-5120.
Moore, M.J. (1995) Nature, 374, 766-7.
Neuberger, M.S. and Milstein, C. (1995). Somatic hypermutation. Curr. Opin. Immunol. 7, 248-254.
Parham, P. (ed). (1998). Somatic hypermutation of immunoglobulin genes. Immunological Reviews, Vol. 162 (Copenhagen, Denmark: Munksgaard).
Ratech, H. (1992). Rapid cloning of rearranged immunoglobulin heavy chain genes from human B-cell lines using anchored polymerase chain reaction, Biochem. Biophys. Res. Commun. 182, 260-1263
Rogozin, I.B. and Kolchanov, N.A. (1992). Somatic hypermutation of immunoglobulin genes. II. Influence of neighbouring base sequences on mutagenesis. Biochem. Biophys. Acta 1171, 11-18.
Sharpe et al., (1991) EMBO J. 10:2139-2145,
Smith, G.P. (1985) Science, 228, 1315-7. Tomlinson, I.M. (1997). V Base database of human antibody genes. Medical Research
Council, Centre for Protein Engineering, UK, http://www.mrc-cpe.cam.ac.uk/ Tuerk, C. and Gold, L, (1990) Science, 249, 505-10.
Wabl, M., Burrows, P.D., von Gabain, A. and Steinberg, C.M. (1985). Hypermutation at the immunoglobulin heavy chain locus in a pre-B-cell line. Proc. Natl. Acad. Sci. USA 82, 479-482.
Wagner, SD, Milstein, C. and Neuberger, M.S. (1995) Codon bias targets mutation Nature 376, 732.
Weill, J.-C. and Reynaud, C.-A. (1996). Rearrangement/hypermutation/gene conversion: when, where and why? Immunol. Today 17, 92-97.
Wilson, P.C., de Boutellier, O., Liu, Y-J., Potter, K.., Banchereau, J., Capra, J.D. and Pascual, V. (1998). Somatic hypermutation introduces insertions and deletions into immunoglobulin V genes. J. Exp. Med. 187, 59-70
Wu, H., Pelkonen, E, Knuutila, S and Kaartinen M. (1995). A human follicular lymphoma B cell line hypermutates its functional immunoglobulin genes in vitro. Eur. J. Immunol, 25, 3263-3269.
Wu, H, and Kaartinen, M., (1995) Scand. J. Immunol. 42:52-59.
Zhu, M., Rabinowitz, J.L., Green, N.S., Kobrin, B.J. and Scharff, M.D. (1995) A well-differentiated B cell line is permissive for somatic mutation of a transfected immunoglobulin heavy-chain gene. Proc. Natl. Acad. Sci. USA 92, 2810-2814,
Zou, X., Xian, J., Popov, A. V., Rosewell, I. R., Muller, M. and Brüggemann, M., Eur, J. Immunol. 1995. 25: 2154-62.

## Claims

1. Use of an immunoglobulin-expressing eukaryotic cell in an *in vitro* method of directed constitutive hypermutation of one or more nucleic acid sequences in the preparation and isolation of a gene product having a desired activity, wherein the one or more nucleic acid sequences encoding the gene product are operatively linked to control sequences which direct somatic hypermutation of the one or more nucleic acids encoding the gene product without the requirement for external stimulation to produce one or more mutated nucleic acid sequences, wherein the one or more mutated nucleic acid sequences encode a gene product having a desired activity.

2. Use according to claim 1, wherein the immunoglobulin-expressing eukaryotic cell is used to produce a population of cells expressing mutants of the gene product.

3. Use according to claim 2, wherein a cell or cells within the population of cells expressing mutants of the gene product are identified.

4. Use according to any one of claims 1-3, wherein the cell or cells direct somatic hypermutation to an endogenous V gene locus.

5. Use according to any one of claims 1-4, wherein the control sequences which direct hypermutation are selected from sequences occurring downstream of a J gene cluster.

6. Use according to claim 5, wherein the control sequences comprise elements Ei/MAR, Cκ plus flanking regions, and the enhancer E3'.

7. Use according to any one of claims 1-6, wherein the one or more nucleic acid sequences operatively linked to control sequences which direct somatic hypermutation is an exogenous nucleic acid sequence inserted into the cell or cells.

8. Use according to claim 7, wherein the exogenous nucleic acid sequence comprises a heterologous coding sequence operably linked to control sequences which direct somatic hypermutation, wherein the control sequences are homologous to the cell or cells.

9. Use according to claim 8, wherein an endogenous V region coding sequence is replaced by the exogenous nucleic acid sequence.

10. Use according to any one of claims 1-9, wherein the gene product is an immunoglobulin heavy chain and/or an immunoglobulin light chain.

11. Use according to any one of claims 1-9, wherein the gene product is a DNA binding protein.

12. Use according to any one of claims 1-9, wherein the gene product is an enzyme.

13. Use according to any one of claims 1-12, wherein the desired activity is a binding activity or enzymatic activity.

14. Use according to claim 10, wherein the gene product is an immunoglobulin heavy chain or an immunoglobulin light chain having an altered binding specificity or an altered binding affinity.

15. Use according to claim 14, wherein the immunoglobulin is an IgM molecule.

16. Use according to any one of claims 1-15, wherein the immunoglobulin-expressing eukaryotic cell is capable of altering one or more specific sections of the one or more nucleic acid sequences without the requirement for external stimulation.

17. Use according to any one of claims 1-16, wherein the immunoglobulin-expressing eukaryotic cell is derived from a cell type which hypermutates *in vivo.*

18. Use according to claim 17, wherein the immunoglobulin-expressing eukaryotic cell is derived from a Burkitt lymphoma cell, a follicular lymphoma cell, or a diffuse large cell lymphoma cell line.

## Patentansprüche

1. Verwendung einer Immunglobulin exprimierenden eukaryontischen Zelle bei einem In-vitro-Verfahren zur gelenkten konstitutiven Hypermutation einer oder mehrerer Nukleinsäuresequenzen bei der Herstellung und Isolierung eines Genprodukts mit einer gewünschten Aktivität, wobei die eine oder mehreren das Genprodukt codierenden Nukleinsäuresequenzen in operativer Verknüpfung mit Kontrollsequenzen stehen, die eine somatische Hypermutation der einen oder mehreren das Genprodukt codierenden Nukleinsäuren ohne Notwendigkeit für eine externe Stimulierung lenken, so dass eine oder mehrere mutierte Nukleinsäuresequenzen produziert werden, wobei die eine oder mehreren mutierten Nukleinsäuresequenzen ein Genprodukt mit einer gewünschten Aktivität codieren.

2. Verwendung nach Anspruch 1, wobei die Immunglobulin exprimierende eukaryontische Zelle verwendet wird, eine Mutanten des Genprodukts exprimierende Population von Zellen zu produzieren.

3. Verwendung nach Anspruch 2, wobei eine Zelle oder Zellen innerhalb der Mutanten des Genprodukts exprimierenden Population von Zellen identifiziert wird bzw. werden.

4. Verwendung nach einem der Ansprüche 1-3, wobei die Zelle bzw. Zellen somatische Hypermutation auf einen endogenen V-Gen-Locus lenken.

5. Verwendung nach einem der Ansprüche 1-4, wobei die Kontrollsequenzen, die die Hypermutation lenken, aus Sequenzen ausgewählt sind, die stromabwärts von einem J-Gen-Cluster auftreten.

6. Verwendung nach Anspruch 5, wobei die Kontrollsequenzen Elemente Ei/MAR, Cκ plus flankierende Bereiche und den Enhancer E3' umfassen.

7. Verwendung nach einem der Ansprüche 1-6, wobei es sich bei der einen bzw. den mehreren Nukleinsäuresequenzen in operativer Verknüpfung mit Kontrollsequenzen, die somatische Hypermutation lenken, um eine in die Zelle oder Zellen inserierte exogene Nukleinsäuresequenz handelt.

8. Verwendung nach Anspruch 7, wobei die exogene Nukleinsäuresequenz eine heterologe Codiersequenz in operativer Verknüpfung mit Kontrollsequenzen, die somatische Hypermutation lenken, umfasst, wobei die Kontrollsequenzen für die Zelle bzw. Zellen homolog sind.

9. Verwendung nach Anspruch 8, wobei eine endogene V-Region-Codiersequenz durch die exogene Nukleinsäuresequenz ersetzt wird.

10. Verwendung nach einem der Ansprüche 1-9, wobei es sich bei dem Genprodukt um eine Immunglobulin-schwere-Kette und/oder eine Immunglobulin-leichte-Kette handelt.

11. Verwendung nach einem der Ansprüche 1-9, wobei es sich bei dem Genprodukt um ein DNA bindendes Protein handelt.

12. Verwendung nach einem der Ansprüche 1-9, wobei es sich bei dem Genprodukt um ein Enzym handelt.

13. Verwendung nach einem der Ansprüche 1-12, wobei es sich bei der gewünschten Aktivität um eine Bindungsaktivität oder enzymatische Aktivität handelt.

14. Verwendung nach Anspruch 10, wobei es sich bei dem Genprodukt um eine Immunglobulin-schwere-Kette oder eine Immunglobulin-leichte-Kette mit einer veränderten Bindungsspezifität oder einer veränderten Bindungsaffinität handelt.

15. Verwendung nach Anspruch 14, wobei es sich bei dem Immunglobulin um ein IgM-Molekül handelt.

16. Verwendung nach einem der Ansprüche 1-15, wobei die Immunglobulin exprimierende eukaryontische Zelle in der Lage ist, einen oder mehrere spezifische Abschnitte der einen oder mehreren Nukleinsäuresequenzen ohne die Notwendigkeit für eine externe Stimulierung zu verändern.

17. Verwendung nach einem der Ansprüche 1-16, wobei die Immunglobulin exprimierende eukaryontische Zelle von einem Zelltyp abgeleitet ist, der in vivo hypermutiert.

18. Verwendung nach Anspruch 17, wobei die Immunglobulin exprimierende eukaryontische Zelle von einer Burkitt-Lymphom-Zelle, einer Follikuläres-Lymphom-Zelle oder einer Diffuses-großzelliges-Lymphom-Zelllinie abgeleitet ist.

## Revendications

1. Utilisation d'une cellule eucaryote exprimant les immunoglobulines dans un procédé *in vitro* d'hypermutation constitutive dirigée d'une ou plusieurs séquences d'acides nucléiques lors de la préparation et de l'isolement d'un produit génique ayant une activité souhaitée, la ou les séquences d'acides nucléiques qui codent pour le produit génique étant fonctionnellement liées pour contrôler les séquences qui dirigent l'hypermutation somatique du ou des acides nucléiques qui codent pour le produit génique, sans exiger une stimulation externe pour produire une ou plusieurs séquences d'acides nucléiques mutées, la ou les séquences d'acides nucléiques mutées codant pour un produit génique ayant une activité souhaitée.

2. Utilisation selon la revendication 1, pour laquelle la cellule eucaryote exprimant les immunoglobulines est utilisée pour produire une population de cellules exprimant des mutants du produit génique.

3. Utilisation selon la revendication 2, pour laquelle une cellule ou des cellules au sein de la population de cellules exprimant des mutants du produit génique sont identifiées.

4. Utilisation selon l'une quelconque des revendications 1 à 3, pour laquelle la cellule ou les cellules dirigent une hypermutation somatique vers un locus de gène V endogène.

5. Utilisation selon l'une quelconque des revendications 1 à 4, pour laquelle les séquences de contrôle qui dirigent l'hypermutation sont choisies parmi les séquences apparaissant en aval d'une batterie de gènes J.

6. Utilisation selon la revendication 5, pour laquelle les séquences de contrôle comprennent les éléments Ei/MAR, les régions flanquant Cκ plus, et l'amplificateur E3'.

7. Utilisation selon l'une quelconque des revendications 1 à 6, pour laquelle la ou les séquences d'acides nucléiques fonctionnellement liées à des séquences de contrôle qui dirigent une hypermutation somatique sont une séquence d'acide nucléique exogène insérée dans la ou les cellules.

8. Utilisation selon la revendication 7, pour laquelle la séquence d'acide nucléique exogène comprend une séquence codante hétérologue fonctionnellement liée pour contrôler les séquences qui dirigent une hypermutation somatique, les séquences de contrôle étant homologues de la cellule ou des cellules.

9. Utilisation selon la revendication 8, pour laquelle une séquence codant pour une région V endogène est remplacée par la séquence d'acide nucléique exogène.

10. Utilisation selon l'une quelconque des revendications 1 à 9, pour laquelle le produit génique est une chaîne lourde d'immunoglobuline et/ou une chaîne légère d'immunoglobuline.

11. Utilisation selon l'une quelconque des revendications 1 à 9, pour laquelle le produit génique est une protéine de liaison à l'ADN.

12. Utilisation selon l'une quelconque des revendications 1 à 9, pour laquelle le produit génique est une enzyme.

13. Utilisation selon l'une quelconque des revendications 1 à 12, pour laquelle l'activité souhaitée est une activité de liaison ou une activité enzymatique.

14. Utilisation selon la revendication 10, pour laquelle le produit génique est une chaîne lourde d'immunoglobuline et une chaîne légère d'immunoglobuline ayant une spécificité de liaison altérée et une affinité de liaison altérée.

15. Utilisation selon la revendication 14, pour laquelle l'immunoglobuline est une molécule d'IgM.

16. Utilisation selon l'une quelconque des revendications 1 à 15, pour laquelle la cellule eucaryote exprimant les immunoglobulines est capable d'altérer une ou plusieurs sections spécifiques de la ou des séquences d'acides nucléiques, sans exiger de stimulation externe.

17. Utilisation selon l'une quelconque des revendications 1 à 16, pour laquelle la cellule eucaryote exprimant les immunoglobulines dérive d'un type cellulaire qui subit une hypermutation *in vivo.*

18. Utilisation selon la revendication 17, pour laquelle la cellule eucaryote exprimant les immunoglobulines dérive d'une cellule de lymphome de Burkitt, d'une cellule de lymphome folliculaire ou d'une lignée de cellules de lymphome à grandes cellules diffuses.
